(19) Europäisches Patentamt
European Patent Office
Office européen des brevets

(11) Numéro de publication: **0 201 400**
**B1**

(12) # FASCICULE DE BREVET EUROPEEN

(45) Date de publication du fascicule du brevet: **19.07.89**

(51) Int. Cl.⁴: **C07D 209/08**, C07D 209/34, A61K 31/40

(21) Numéro de dépôt: **86400884.2**

(22) Date de dépôt: **23.04.86**

(54) **Dérivés du 4-phénylpropyl indole et leurs sels, leur procédé de préparation, leur application à titre de médicaments et les compositions pharmaceutiques les renfermant.**

(30) Priorité: **23.04.85 FR 8506135**
**21.01.86 FR 8600761**

(43) Date de publication de la demande:
**17.12.86 Bulletin 86/46**

(45) Mention de la délivrance du brevet:
**19.07.89 Bulletin 89/29**

(84) Etats contractants désignés:
**AT BE CH DE GB IT LI LU NL SE**

(56) Documents cités:
**EP-A- 0 099 766**

(73) Titulaire: **ROUSSEL-UCLAF, 35, boulevard des Invalides, F-75007 Paris(FR)**

(72) Inventeur: **Clémence, François, 2, rue Turgot, F-75009 Paris(FR)**
Inventeur: **Guillaume, Jacques, 62, rue Pexerecourt, F-75020 Paris(FR)**
Inventeur: **Hamon, Gilles, 40, rue de Bagneux, F-92120 Montrouge(FR)**

(74) Mandataire: **Tonnellier, Marie-José et al, Département des Brevets ROUSSEL UCLAF B.P. no 9, F-93230 Romainville(FR)**

EP 0 201 400 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**Description**

La présente invention concerne des dérivés du 4-phénylpropyl-indole ainsi que leurs sels, leur procédé de préparation, leur application à titre de médicaments et les compositions pharmaceutiques les renfermant.

L'invention a pour objet des dérivés du 4-phénylpropyl-indole, ainsi que leurs sels d'addition avec les acides minéraux ou organiques, caractérisés en ce qu'ils répondent à la formule générale I:

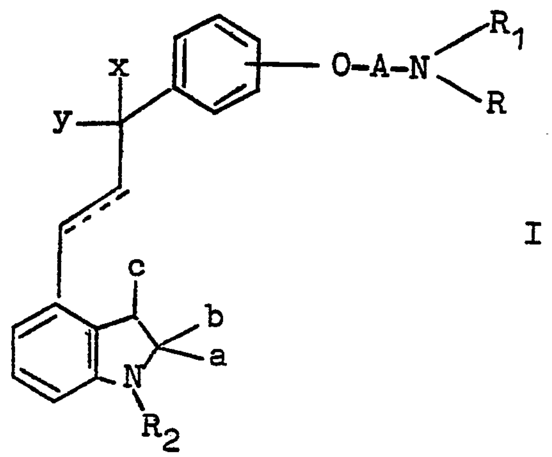

I

dans laquelle R et $R_1$ représentent, indépendamment l'un de l'autre, un atome d'hydrogène, un radical alkyle linéaire renfermant de 1 à 5 atomes de carbone, un radical alkyle ramifié renfermant de 3 à 5 atomes de carbone, un radical cycloalkyle renfermant de 3 à 7 atomes de carbone, un radical cycloalkylalkyle renfermant de 4 à 7 atomes de carbone, ou un radical aralkyle renfermant de 7 à 12 atomes de carbone ou un radical aralkyle renfermant de 7 à 12 atomes de carbone substitué, choisi parmi les radicaux benzyle ou phénétyle substitués par 1, 2 ou 3 radicaux choisis dans le groupe constitué par les halogènes et les radicaux méthyle, éthyle, méthoxy, éthoxy, trifluorométhyle, méthylthio, amino et nitro, ou R et $R_1$ forment ensemble avec l'atome d'azote un cycle pyrrolidino, pipéridino, morpholino, pipérazinyle, méthyl pipérazinyle, éthyl pipérazinyle, propyl pipérazinyle, a représente ensemble avec b une fonction oxo, ou représente ensemble avec c une liaison carbone-carbone, b représente un atome d'hydrogène, ou ensemble avec a une fonction oxo, c représente un atome d'hydrogène, ou ensemble avec a représente une liaison carbone-carbone, le pointillé représente la présence éventuelle d'une liaison carbone-carbone, A représente une chaîne $-(CH_2)_n-$ dans laquelle n peut prendre les valeurs 2, 3, 4 ou 5, $R_2$ représente un atome d'hydrogène, un radical alkyle linéaire renfermant de 1 à 5 atomes de carbone ou un radical alkyle ramifié renfermant de 3 à 5 atomes de carbone, x représente un radical hydroxyle, ou un atome d'hydrogène, ou ensemble avec y, une fonction oxo et y représente un atome d'hydrogène ou, ensemble avec x, une fonction oxo.

Dans la formule générale (I) et dans ce qui suit, le terme radical alkyle linéaire renfermant de 1 à 5 atomes de carbone désigne, de préférence, un radical méthyle, éthyle ou propyle; le terme radical alkyle ramifié renfermant de 3 à 5 atomes de carbone désigne, de préférence, un radical isopropyle ou tert-butyle, le terme cycloalkyle renfermant de 3 à 7 atomes de carbone désigne, de préférence, un radical cyclopentyle; le terme radical cycloalkylalkyle renfermant de 4 à 7 atomes de carbone désigne, de préférence, un radical cyclopropylméthyle; le terme radical aralkyle renfermant de 7 à 12 atomes de carbone désigne, de préférence, un radical benzyle ou phénéthyle.

Les sels d'addition avec les acides minéraux ou organiques peuvent être, par exemple, les sels formés avec les acides chlorhydrique, bromhydrique, nitrique, sulfurique, phosphorique, acétique, formique, propionique, benzoïque, maléique, fumarique, succinique, tartrique, citrique, oxalique, glyoxylique, aspartique, alcane sulfoniques tels que les acides méthane ou éthane sulfoniques, arylsulfoniques, tels que les acides benzène ou paratoluène sulfoniques et arylcarboxyliques.

Parmi les produits objet de l'invention, on peut citer notamment les dérivés répondant à la formule (I) ci-dessus, ainsi que leurs sels d'addition avec les acides minéraux ou organiques, caractérisés en ce que, dans ladite formule (I), a et c représentent ensemble une liaison carbone-carbone.

Parmi ceux-ci, on peut citer plus particulièrement les dérivés caractérisés en ce que dans ladite formule (I), $R_1$ et $R_2$ représentent un atome d'hydrogène et tout particulièrement :

la N-/2-/2-/3-(1H-indol-4-yl) propyl/phénoxy/éthyl/2-méthyl-2-propanamine,

la 1-/2-/3-/(1,1-diméthyl éthyl) amino/propoxy/phényl/3-(1H-indol-4-yl) propanone et,

la N-/2-/2-/3-(1H-indol-4-yl)propyl/phénoxy/éthyl/N-(1-méthyl) 2-propanamine,

ainsi que leurs sels d'addition avec les acides minéraux ou organiques.

On peut citer également les produits décrits dans les exemples ainsi que ceux répondant à la formule:

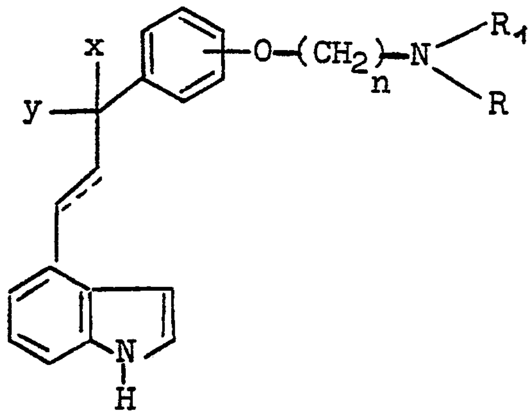

dans laquelle les différents paramètres ont les valeurs suivantes:

| pointillé | x | y | O- | n | R | $R_1$ |
|---|---|---|---|---|---|---|
| 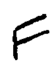 | = | 0 | 3 | 2 | méthyl | méthyl |
| 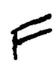 | = | 0 | 4 | 2 | " | " |
| 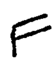 | = | 0 | 2 | 3 | " | " |
| 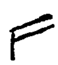 | = | 0 | 3 | 3 | " | " |
| 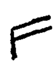 | = | 0 | 4 | 3 | " | " |
| - | OH | H | 3 | 2 | " | " |
| - | OH | H | 4 | 2 | " | " |
| - | OH | H | 2 | 3 | " | " |

| pointillé | x | y | O- | n | R | $R_1$ |
|---|---|---|---|---|---|---|
| - | OH | H | 3 | 3 | méthyl | méthyl |
| - | OH | H | 4 | 3 | " | " |
| - | H | H | 3 | 2 | " | " |
| - | H | H | 4 | 2 | " | " |
| - | H | H | 2 | 3 | " | " |
| - | H | H | 3 | 3 | " | " |
| - | H | H | 4 | 3 | " | " |
| - | OH | H | 4 | 2 | t-butyl | H |
| - | H | H | 4 | 2 | " | " |
| 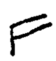 | OH | H | 4 | 2 | " | " |
| 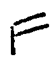 | = | 0 | 3 | 2 | 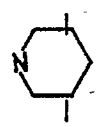 | |
| 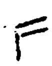 | = | 0 | 4 | 2 | " | " |
| 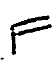 | = | 0 | 2 | 3 | " | " |
| 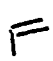 | = | 0 | 3 | 3 | " | " |
| 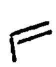 | = | 0 | 4 | 3 | " | " |

| pointillé | x | y | O- | n | R | $R_1$ |
|---|---|---|---|---|---|---|
| - | OH | H | 3 | 2 | 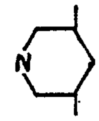 | |
| - | OH | H | 4 | 2 | " | " |
| - | OH | H | 2 | 3 | " | " |
| - | OH | H | 3 | 3 | " | " |
| - | OH | H | 4 | 3 | " | " |
| - | H | H | 3 | 2 | " | " |
| - | H | H | 4 | 2 | " | " |
| - | H | H | 2 | 3 | " | " |
| - | H | H | 3 | 3 | " | " |
| | H | H | 4 | 3 | " | |
| ╒ | = | 0 | 3 | 2 | isopropyl | isopropyl |
| ╒ | = | 0 | 4 | 2 | " | " |
| ╒ | = | 0 | 2 | 3 | " | " |
| ╒ | = | 0 | 3 | 3 | " | " |

| pointillé | x | y | O- | n | R | $R_1$ |
|---|---|---|---|---|---|---|
| | | =0 | 4 | 3 | isopropyl | isopropyl |
| - | OH | H | 3 | 2 | " | " |
| - | OH | H | 4 | 2 | " | " |
| - | OH | H | 2 | 3 | " | " |
| - | OH | H | 3 | 3 | " | " |
| - | OH | H | 4 | 3 | " | " |
| - | H | H | 3 | 2 | " | " |
| - | H | H | 4 | 2 | " " | " |
| - | H | H | 2 | 3 | " " | " |
| - | H | H | 3 | 3 | isopropyl | isopropyl |
| - | H | H | 4 | 3 | " | " |
| - | | =0 | 2 | 2 | propyl | H |
| - | H | H | 2 | 2 | " | H |

ainsi que les dérivés 2-oxo correspondants.

L'invention a également pour objet un procédé de préparation des dérivés, tels que définis par la formule I ci-dessus, ainsi que de leurs sels, caractérisé en ce que l'on fait réagir un dérivé de formule II :

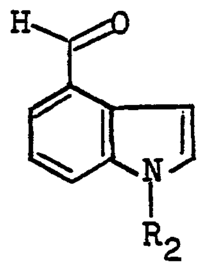

II

dans laquelle $R_2$ a la signification déjà indiquée, avec un dérivé de formule III :

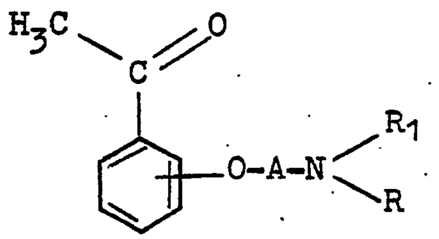

III

dans laquelle R et $R_1$ ont la signification déjà indiquée, pour obtenir un produit de formule $I_A$ :

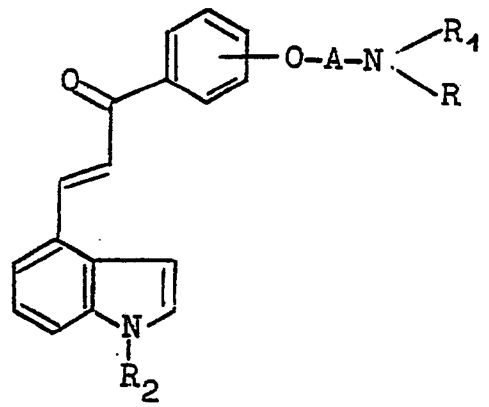

$I_A$

dans laquelle R, $R_1$ et $R_2$ ont la signification déjà indiquée, que soit l'on hydrogène par action d'hydrogène gazeux en présence d'un catalyseur à base de platine ou de palladium dans un solvant tel qu'un alcanol renfermant de 1 à 5 atomes de carbone, ou par action d'hydrogène gazeux en présence de Nickel de Raney dans un solvant tel que l'acétate d'éthyle, ou par action pendant moins de 3 heures, de sodium dans l'ammoniac, dans un solvant tel que le tétrahydrofuranne, pour obtenir un produit de formule $I_B$:

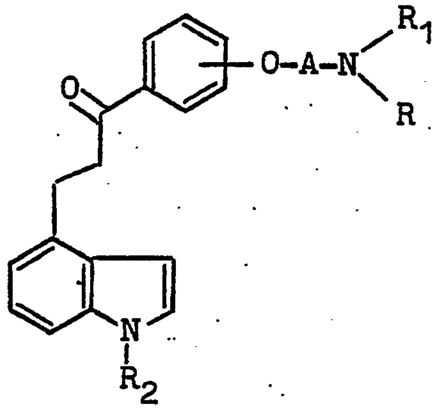

$I_B$

dans laquelle A, R, $R_1$ et $R_2$ ont la signification déjà indiquée, que ou bien l'on isole et, si désiré, salifie, ou bien l'on réduit à l'aide d'un borohydrure ou cyanoborohydrure alcalin pour obtenir un produit de formule $I_C$:

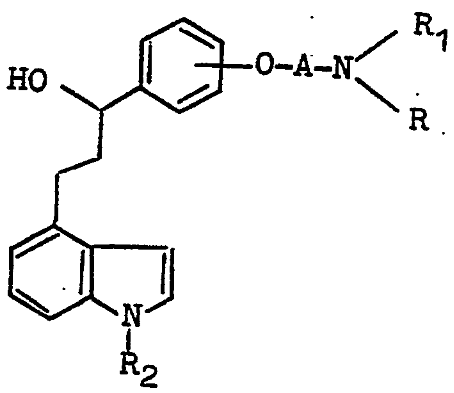

$I_C$

dans laquelle A, R, $R_1$ et $R_2$ ont la signification déjà indiquée, que on bien l'on isole et, si désiré, salifie, ou bien l'on réduit à l'aide de sodium dans l'ammoniac pour obtenir un produit de formule $I_D$:

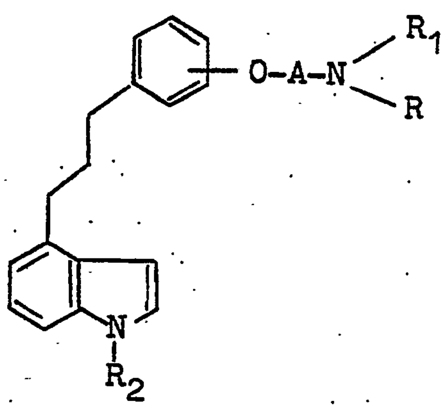

$I_D$

dans laquelle A, R, $R_1$ et $R_2$ ont la signification déjà indiquée, que l'on isole, et, si désiré, salifie, soit l'on fait réagir ledit produit de formule $I_A$ avec le complexe formé entre un borohydrure alcalin et la pyridine, pour obtenir un produit de formule $I_E$:

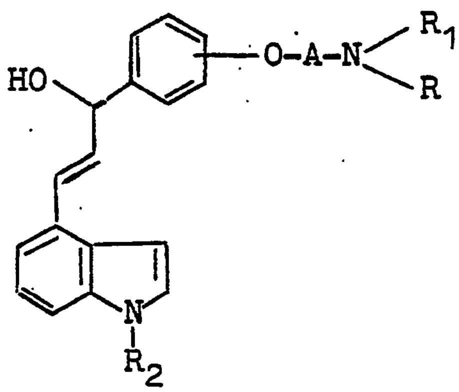

$I_E$

dans laquelle A, R, $R_1$ et $R_2$ ont la signification déjà indiquée, que ou bien l'on isole, ou bien l'on réduit à l'aide de sodium dans l'ammoniaque pour obtenir un produit de formule $I_D$ que l'on isole et, si désiré, salifie,

soit l'on réduit ledit produit de formule $I_A$ à l'aide de sodium dans l'ammoniaque pour obtenir un produit de formule $I_D$ que l'on isole et, si désiré salifie,

puis soumet, si désiré, lesdits produits de formule :

$I_A$, $I_B$, $I_C$, $I_D$ et $I_E$ à l'action d'un agent d'halogénation pour obtenir un produit de formule IV :

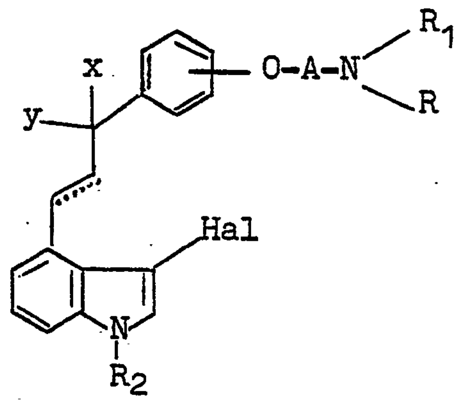

IV

dans laquelle Hal représente un atome de brome ou de chlore, et x, y, A, R, $R_1$, $R_2$ et le pointillé ont la signification déjà indiquée, que l'on soumet à une hydrolyse pour obtenir un produit de formule $I_F$:

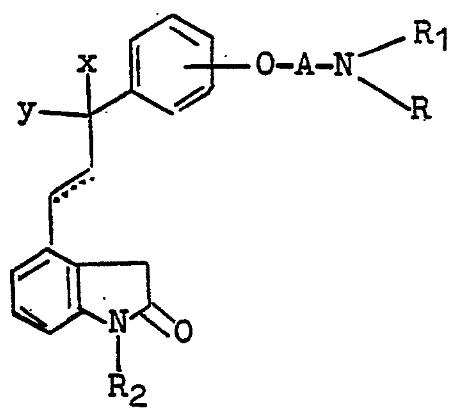

$I_F$

dans laquelle x, y, A, R, $R_1$, $R_2$ et le pointillé ont la signification déjà indiquée, que l'on isole et, si désiré, salifie.

La réaction du 4-formyl indole de formule II avec le dérivé de formule III est réalisée de préférence en présence d'une base minérale telle que l'hydroxyde de sodium ou de potassium, par exemple au sein d'un solvant tel qu'un alcanol renfermant de 1 à 5 atomes de carbone, notamment l'éthanol.

Lorsque l'on veut préparer un produit de formule $I_B$ à partir d'un produit de formule $I_A$ par action de

sodium dans l'ammoniac, le temps de contact est de préférence d'environ 1 heure ; lorsque l'on veut préparer un produit de formule $I_D$, le temps de contact est de préférence d'environ 6 heures. On opère de préférence en présence d'un solvant tel que le tétrahydrofuranne.

Le borohydrure ou cyanoborohydrure alcalin utilisé pour la réduction du produit de formule $I_B$ est par exemple le cyanoborohydrure de potassium ou de sodium ou de préférence le borohydrure de sodium. La réduction du produit de formule $I_B$ peut aussi être réalisée à l'aide d'hydrazine et de lessive de potasse dans l'éthylène glycol.

Le borohydure alcalin utilisé en complexe avec la pyridine est de préférence celui de sodium, on le fait réagir de préférence dans un alcanol renfermant au plus 5 atomes de carbone, tel que l'éthanol.

L'halogénation des produits de formules ($I_A$), ($I_B$), ($I_C$), ($I_D$) et ($I_E$) peut être réalisée, par exemple, à l'aide du complexe bromé de la pyridine de formule:

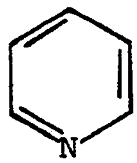

$Br_2$, HBr

dans le cas de la bromation. Elle est réalisée avantageusement à l'aide d'un N-halo succinimide, de préférence le N-bromo ou le N-chloro succinimide : on opère dans le dioxanne ou de préférence dans l'acide acétique. Le produit de formule (IV) obtenu est de préférence un produit chloré.

L'hydrolyze du produit de formule (IV) est réalisée, de préférence à l'aide d'un acide minéral tel que l'acide phosphorique, l'acide sulfurique, ou de préférence l'acide chlorhydrique en solution aqueuse. Cette solution peut être utilisée concentrée, mais de préférence diluée par exemple en solution normale. On peut utiliser, en outre, un solvant tel qu'un alcool aliphatique comme l'éthanol.

L'invention a également pour objet un procédé pour la préparation des dérivés répondant à la formule I et de leurs sels, caractérisé en ce que l'on fait réagir un produit de formule II:

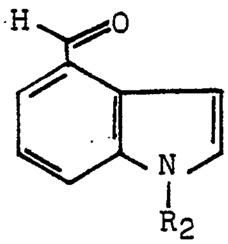

II

dans laquelle $R_2$ a la signification indiquée précédemment avec un dérivé de formule III':

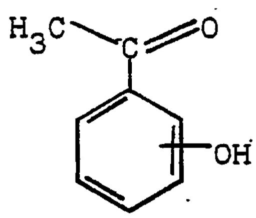

III'

pour obtenir un produit de formule IX:

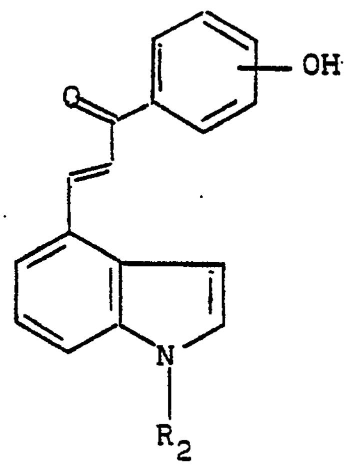

IX

dans laquelle $R_2$ a la signification déjà indiquée, que soit l'on hydrogène par action d'hydrogène gazeux en présence d'un catalyseur à base de platine ou de palladium dans un solvant tel qu'un alcanol renfermant de 1 à 5 atomes de carbone, ou par action d'hydrogène gazeux en présence de Nickel de Raney dans un solvant tel que l'acétate d'éthyle, ou par action pendant moins de 3 heures de sodium dans l'ammoniac dans un solvant tel que le tétrahydrofuranne, pour obtenir un produit de formule X:

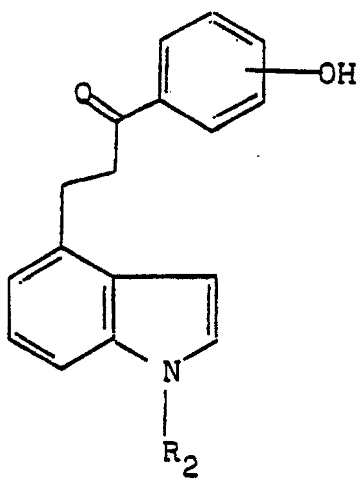

X

dans laquelle $R_2$ a signification déjà indiquée,
que ou bien l'on réduit à l'aide d'un borohydrure ou cyanoborohydrure alcalin pour obtenir un produit de formule XI :

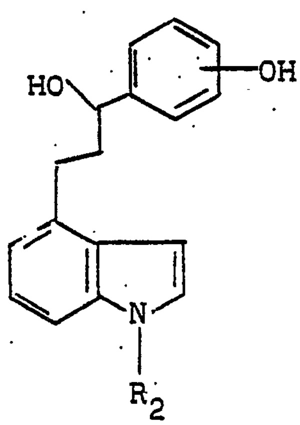

XI

dans laquelle $R_2$ a la signification déjà indiquée, que l'on réduit à l'aide de sodium dans l'ammoniac pour

obtenir un produit de formule XII :

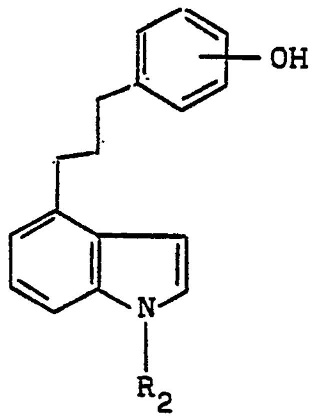

XII

dans laquelle $R_2$ a la signification déjà indiquée, soit l'on fait réagir ledit produit de formule IX avec le complexe formé entre un borohydrure alcalin et la pyridine, pour obtenir un produit de formule XIII:

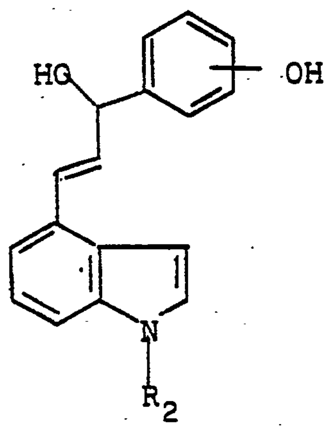

XIII

dans laquelle $R_2$ a la signification déjà indiquée que l'on réduit à l'aide de sodium dans l'ammoniac pour obtenir un produit de formule XII, soit l'on réduit ledit produit de formule IX à l'aide de sodium dans l'ammoniac pour obtenir un produit de formule XII défini ci-dessus ou bien l'on réduit ledit produit de formule X à l'aide d'hydrazine et de lessive de potasse dans l'éthylène glycol, pour obtenir un produit de formule XII défine ci-dessus, puis soumet si désiré lesdits produits de formules IX, X, XI, XII et XIII à l'action d'un agent d'halogénation pour obtenir un produit de formule XIV :

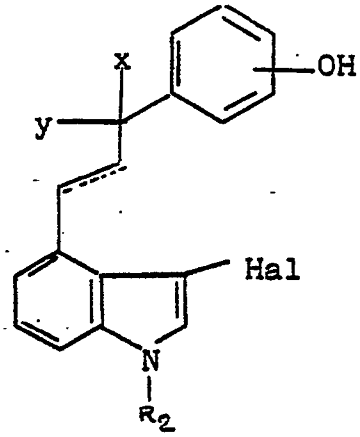

XIV

dans laquelle Hal représente un atome de brome ou de chlore et x, y, $R_2$ et le pointillé ont la signification déjà indiquée, que l'on soumet à une hydrolyse pour obtenir un produit de formule XV :

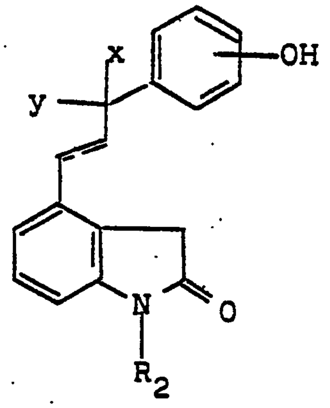

XV

dans laquelle x, y, $R_2$ et le pointillé ont la signification déjà indiquée puis fait réagir lesdits produits de formules IX, X, XI, XII, XIII et XV avec un halogénure de formule XVI :

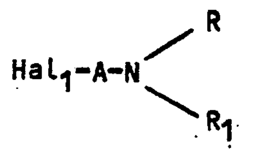

XVI

dans laquelle $Hal_1$ représente un atome de chlore, de brome ou d'iode et A, R et $R_1$ ont la signification indiquée précédemment, pour obtenir un produit de formule $I_F$ :

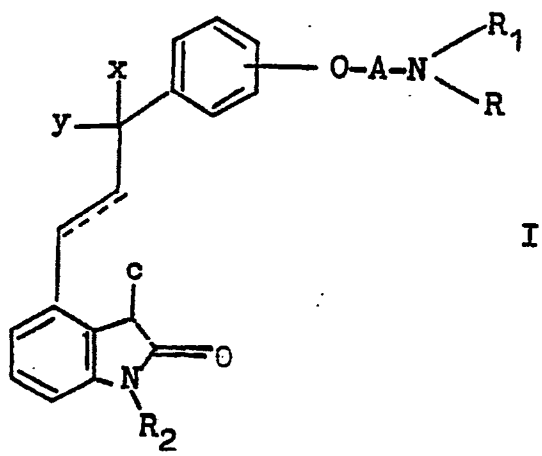

$I_F$

dans laquelle le pointillé, x, y, A, R, $R_1$ et $R_2$ ont la signification déjà indiquée, que l'on isole et, si désiré, salifie.

La réaction du 4-formyl indole de formule II avec le dérivé de formule III' est réalisée de préférence comme indiqué ci-dessus pour la réaction du produit de formule II avec le dérivé de formule III.

Lorsque l'on veut préparer un produit de formule X ou un produit de formule XII, on opère selon les conditions indiquées ci-dessus pour la préparation des produits de formule $I_B$ ou $I_D$.

Le borohydrure ou cyanoborohydrure alcalin utilisé pour la réduction du produit de formule X est par exemple le cyanoborohydrure de potassium ou de sodium ou de préférence le borohydrure de sodium et l'hydrazine est utilisée de préférence sous forme d'hydrate en présence de potasse et dans l'éthylène-glycol.

Le borohydrure alcalin utilisé en complexe avec la pyridine est de préférence celui de sodium, on le fait réagir de préférence dans un alcanol renfermant au plus 5 atomes de carbone, tel que l'éthanol.

L'halogénation des produits des formules IX, X, XII et XIII peut être réalisée comme indiqué ci-dessus pour l'halogénation des produits de formules $I_A$, $I_B$, $I_C$, $I_D$ et $I_E$.

Le produit de formule XIV obtenu est de préférence un produit chloré.

L'hydrolyse du produit de formule XIV est réalisée de manière identique à celle du produit de formule IV.

La réaction du produit de formule XV avec L'halogénure de formule XVI est réalisée par réaction de

transfert de phase en utilisant comme phase aqueuse de préférence une solution aqueuse d'un hydroxyde alcalin tel que l'hydroxyde de sodium et comme phase organique non miscible à l'eau un solvant tel que le benzène, en opérant en présence d'un agent de transfert tel que le bromure ou de préférence l'hydrogénosulfate de tétrobutylammonium.

Les produits de formule II sont connus ou peuvent être préparés comme indiqué, notamment dans J. Org. Chem. 1980, 45 p.3350 et suivantes.

Les produits de formule III' sont également connus.

Les produits de formule III sont connus ou peuvent être préparés comme indiqué notamment dan Helv. Chim. Acta 46 1696-704 (1963). Ils peuvent aussi être préparés, par exemple, par réaction de transfert de phase entre un dérivé du phénol de formule V :

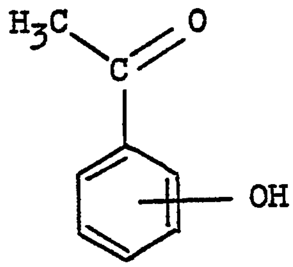

V

et soit un w-chloralkyl paratoluène sulfonate, en utilisant comme phase aqueuse de préférence une solution aqueuse d'un hydroxyde alcalin tel que d'hydroxyde de sodium et comme phase organique non miscibleà l'eau un solvant tel que le benzène, en présence d'un agent de transfert tel que le bromure ou l'hydrogénsulfate de tétrabutylammonium, soit un α-bromo-w-chloro alkylène, de préférence, en présence d'un agent de condensation tel qu'une base, notamment un carbonate ou un bicarbonate alcalin, pour obtenir un produit de formule VI :

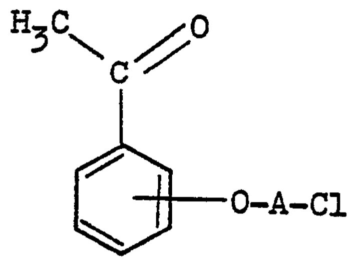

VI

dans laquelle A a la signification déjà indiquée, que l'on fait réagir avec une amine de formule VII :

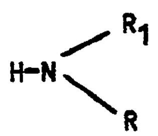

VII

dans laquelle R et $R_1$ ont la signification déjà indiquée, $R_1$ et R ne pouvant représenter en même temps l'hydrogène dans un solvant de préférence en présence d'un agent de condensation, tel qu'un carbonate alalin, ou en utilisant l'amine comme solvant. Pour avoir un produit de formule III dans laquelle R = $R_1$ = H, on fait réagir le dérivé de formule V avec le nitrile de formule VIII :

$Cl-A'-C\equiv N$ VIII

dans laquelle A' est l'homologue inférieur de A, de préférence en présence d'un agent de condensation, puis procède à une hydrogénation catalytique du produit obtenu, de préférence en présence de Nickel de Raney.

Les dérivés de formule (I) présentent un caractère basique. On peut avantageusement préparer les sels d'addition des dérivés de formule (I), en faisant réagir, en proportions sensiblement stoechiométriques, un acide minéral ou organique avec ledit dérivé de formule (I). Les sels peuvent être préparés sans isoler les bases correspondantes.

Les dérivés, objet de la présente invention, possèdent de très intéressantes propriétés pharmacologiques ; ils sont doués notamment de remarquables propriétés anti-arythmiques et bloquantes des canaux calcicosodiques lents. Ces propriétés sont illustrées plus loin dans la partie expérimentale.

Certains possèdent, en outre, des propriétés antisérotoninergiques. Les propriétés pharmacologiques des produits de la présente demande peuvent aussi être mises en évidence à l'aide d'autres tests pharmacologiques décrits antérieurement dans la littérature pour la détection de ces propriétés, par exemple le test d'inhibition du bronchospasme à la sérotonine pour l'activité antisérotoninergique.

Ces propriétés justifient l'utilisation des dérivés du 4-phényl propyl-indole, ainsi que de leurs sels pharmaceutiquement acceptables à titre de médicaments.

La présente demande a ainsi également pour objet l'application, à titre de médicaments, des dérivés du 4-phénylpropyl-indole, tels que définis par la formule I, ainsi que de leurs sels d'addition avec les acides pharmaceutiquement acceptables.

Parmi les médicaments, objet de l'invention, on retient, de préférence, les médicaments caractérisés en ce qu'ils sont constitués par les nouveaux dérivés du 4-phénylpropyl-indole répondant à la formule I, dans laquelle a et c représentent ensemble une liaison carbone-carbone ainsi que par leurs sels d'addition avec les acides pharmaceutiquement acceptables.

Parmi ceux-ci, on retient notamment ceux répondant à la formule I, dans laquelle $R_1$ et $R_2$ représentent un atome d'hydrogène, ainsi que leurs sels d'addition avec les acides pharmaceutiquement acceptables.

Parmi ces derniers, on retient tout particulièrement :
la N-/2-/2-/3-(1 H-indol-4-yl) propyl/phénoxy/éthyl/2-méthyl-2-propanamine, la 1-/2-/3-(1,1-diméthyléthyl)amino/propoxy/phényl/3-(1H-indol-4-yl)1-propanone et la N-/2-/2-/3-(1H-indol-4-yl)propyl/phénoxy/éthyl l/N-(1-méthyléthyl)2-propanamine ainsi que leurs sels d'addition avec les acides pharmaceutiquement acceptables.

Les médicaments selon l'invention trouvent leur emploi, par exemple dans le traitement de l'insuffisance cardiaque, de l'angor sous toutes ses formes et dans le traitement des arythmies. Certains sont de plus utilisables dans le traitement des spasmes.

La dose usuelle, variable selon le dérivé utilisé, le sujet traité et l'affection en cause peut être par exemple de 50 mg à 1 g par jour. Par voie orale, chez l'homme, le dérivé de l'exemple 4 ou 24 peut être administré à la dose quotidienne de 200 mg à 800 mg. par exemple pour le traitement des arythmies ventriculaires, supraventriculaires et jonctionnelles, soit environ de 3 mg à 12 mg par kilogramme de poids corporel.

L'invention a enfin pour objet les compositions pharmaceutiques qui renferment au moins un dérivé précité ou l'un de ses sels d'addition avec les acides pharmaceutiquement acceptables à titre de principe actif.

A titre de médicaments, le dérivés répondant à la formule (I) et leurs sels d'addition avec les acides pharmaceutiquement acceptables peuvent être incorporés dans des compositions pharmaceutiques destinées à la voie digestive ou parentérale.

Ces compositions pharmaceutiques peuvent être, par exemple, solides ou liquides et se présenter sous les formes pharmaceutiques couramment utilisées en médecine humaine, comme par exemple, les comprimés, simples ou dragéifiés, les gélules, les capsules, les granulés, les suppositoires, les préparations injectables ; ellessont préparées selon les méthodes usuelles. Le ou les principes actifs peuvent y être incorporés à des excipients habituellement employés dans ces composition pharmaceutiques, tels que le talc, la gomme arabique, les lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

Les exemples qui suivent illustrent la présente invention, sans toutefois la limiter.

Exemple 1 : 1-/2-/2-/(1,1-diméthyléthyl) amino/ éthoxy/phényl/3-/1 H-indol-4-yl/ 2-propen-1-one(E) et son (±) 2,3-dihydroxy butane dioate acide.

On ajoute sous agitation et atmosphère inerte 3,339 g d'indole-4-carboxaldéhyde et 6 cm3 de soude à 50 % à une solution de 5,4 g de 1-/2-/2(1,1-diméthyléthyl) aminoéthoxy/phényl/éthanone dans 75 cm3 d'éthanol, agite 4 H, dilue à l'eau, extrait à l'acétate d'éthyle, lave à l'eau salée, sèche sur déshydratant, amène à sec sous pression réduite, purifie par chromatographie sur silice (éluant : acétate d'éthyle-triéthylamine 9-1) et obtient 7,41 g du produit attendu sous forme de base.

Formation du (±) 2,3-dihydroxy butane dioate acide

On ajoute à 80° C sous agitation 1,24 g d'acide DL tartrique à une solution de 3 g de base ci-dessus dans 150 cm3 d'isopropanol et 50 cm3 de méthanol, maintient à 80° C pendant 20 mn, évapore le solvant, reprend le résidu par un mélange de 45 cm3 de méthanol et 50 cm3 d'isopropanol en présence d'un peu de pentane et laisse cristalliser.

On filtre en sèche à 80° C sous pression réduite les cristaux obtenus, et obtient en 2 jets 2,83 g du produit attendu F = 172° C.

Analyse (1er jet) : pour $C_{23}H_{26}N_2O_2,C_4H_6O_6$ = 512,564.

| | | | | | | |
|---|---|---|---|---|---|---|
| Calculé | C% | 63,27 | H% | 6,29 | N% | 5,46 |
| Trouvé | | 63,3 | | 6,6 | | 5,5 |

Exemple 2* 1-/2-/2-/(1,1-diméthyléthyl) amino/ éthoxy/phényl 3-/1 H-indol-4-yl/ 1-propanone et son chlorhydrate.

On hydrogène sous agitation 3,218 g de base de l'exemple 1 en présence 1,1 g de charbon palladié à 10 % dans 350 cm3 de méthanol, filtre, amène à sec sous pression réduite, lave avec 7 cm3 d'isopropanol, filtre, sèche sous pression réduite et obtient en 2 jets, 2,35 g de produit attendu. F≃112¤C.

Formation du chlorhydrate :

On ajoute 4 cm3 d'une solution d'acétate d'éthyle chlorhydrique à une suspension de 2,25 g de base ci-dessus dans 70 cm3 d'isopropanol, filtre, sèche à 80¤C et obtient 2,38 g du produit attendu F ≃ 250¤ C.

Analyse : pour $C_{23}H_{28}N_2O_2$, HCl = 400,952

| | | | | | | | | |
|---|---|---|---|---|---|---|---|---|
| Calculé | C% | 68,9 | H% | 7,29 | N% | 6,99 | Cl% | 8,84 |
| Trouvé | | 68,9 | | 7,5 | | 6,9 | | 8,7 |

Exemple 3 : α-/2-/2-/(1,1-diméthyléthyl) amino/ éthoxy/ phényl-1 H-indole-4-propanol et son (E) butène dioate neutre.

On ajoute 0,934 g de borohydrure de sodium à une solution de 3 g de base l'exemple 2 dans 90 cm3 de méthanol, agite sous atmosphère inerte pendant 20 mn, ajoute de l'eau glacée, extrait à l'acétate d'éthyle, lave à l'eau, sèche sur déshydratant, amène à sec, et obtient 2,79 g du produit attendu.

Formation du (E) butène dioate neutre :

On ajoute 0,862 g d'acide fumarique à une solution de 2,73 g de base ci-dessus dans 70 cm3 d'isopropanol, sous agitation, puis ajoute à nouveau 20 cm3 d'isopropanol et amorce la cristallisation. Après 16 H à +4¤C, on filtre, sèche à 80¤ C sous pression réduite, et obtient 2,5 g du produit attendu F ≃ 190¤C.

Analyse : $(C_{23}H_{30}N_2O_2)_2$, $C_4H_4O_4$ = 849,089.

| | | | | | | |
|---|---|---|---|---|---|---|
| Calculé | C% | 70,73 | H% | 7,60 | N% | 6,60 |
| Trouvé | | 70,5 | | 7,8 | | 6,5 |

Exemple 4 : N-/2-/2-/3-(1H-indol-4-yl) propyl/ phénoxy/ éthyl/2-méthyl-2-propanamine.

On ajoute une solution de 31 mg de base de l'exemple 3 dans 2 cm3 de tétrahydrofuranne à 3 cm3 d'ammoniac à -78¤ C, ajoute 100 mg de sodium à -40¤ C sous atmosphère inerte. Après 1 H d'agitation, on ajoute 800 mg de chlorure d'ammonium à -40¤ C, laisse évaporer à température ambiante dilue à l'eau, extrait à l'acétate d'éthyle, lave à l'eau salée, sèche sur déshydratant, filtre, évapore le solvant, ajoute un peu de pentane, filtre, sèche, et obtient 22 mg du produit attendu F ≃ 112¤ C.

Exemple 5 : 1-/2-/3-/(1,1-diméthyléthyl) amino/ propoxy/phényl/3-/1 H-indol-4-yl/-2-propèn-1-one et son (+) 2,3-dihydroxy butane dioate acide

On ajoute 3 cm3 de soude à 50%, puis 1,484 g d'indole-4-carboxaldéhyde à une solution de 2,55 g de 1-/2-/3-/(1,1-diméthyléthyl) amino/ propoxy/ phényl/ éthanone dans 35 cm3 d'éthanol et agite 20 mn sous atmosphère inerte. On dilue à l'eau, extrait à l'acétate d'éthyle, lave à l'eau, sèche sur déshydratant, filtre, amène à sec sous pression réduite, purifie par chromatographie sur silice (éluant : acétate d'éthyle-triéthylamine 9-1) et obtient 2,804 g du produit attendu.

Formation du (±) 2,3-dihydroxy butane dioate acide

On ajoute 1,065 g d'acide DL tartrique à une solution de 2,672 g de base ci-dessus dans 70 cm3 d'isopropanol à 80° C, maintient pendant 25 mn à cette température, amène à sec, empâte avec du pentane ajoute un mélange de méthanol et d'isopropanol, filtre, sèche à 80° C sous pression réduite, et obtient en 2 jets 2,319 g du produit attendu F ≃ 184° C.

| Analyse : | $C_{24}H_{28}N_2O_2, C_4H_6O_6$ = 526,591 | | | | | |
|---|---|---|---|---|---|---|
| Calculé | C % | 63,86 | H % | 6,51 | N% | 5,32 |
| Trouvé | | 63,7 | | 6,7 | | 5,4 |

Préparation de la 1-/2-/3-/(1,1-diméthyléthyl) amino/ propoxy phényl/éthanone.

Stade A : 1-/2-/3-chloropropoxy/ phényl/ éthanone.

On ajoute sous agitation 2 cm3 de 2-hydroxy acétophénone à un mélange de 2,31 g d'hydrogénosulfate de n-tétrabutyl ammonium et de 20 cm3 de soude à 50 % dans 40 cm3 de benzène et 20 cm3 d'acétonitrile. On ajoute 8,26 g de 3-chloro propyl p-tolène sulfonate et chauffe pendant 17 H à 80 °C sous atmosphère inerte. On décante, extrait au benzène, lave à l'eau, sèche sur déshydratant, filtre, amène à sec, chromatographie sur silice (éluant benzène-acétate d'éthyle 95/5) évapore les solvants et obtient 3,38 g de produit attendu.

Stade B : 1-/2-/3-/(1,1-diméthyléthyl) amino/ propoxy/ phényl/éthanone.

On chauffe à 120°C pendant 19 H un mélange de 2,58 g de produit du stade A, 8,3 cm3 de tert-butylamine et 2,17 g de carbonate de potassium dans 7 cm3 de N,N-diméthylformamide sous agitation. On dilue à l'eau, extrait à l'acétate d'éthyle, lave à l'eau, sèche sur déshydratant, évapore le solvant, chromatographie sur silice (éluant : acétate d'éthyle-triéthylamine 9/1) et obtient 2,571 g du produit attendu.

Exemple 6 : 1-/2-/3-/(1,1-diméthyléthyl) amino/ propyloxy/ phényl/3-(1 H-indol-4-yl) 2-propèn-1-ol.

On chauffe à 70° C pendant 15 mn un mélange de 4,38 g comprenant 4,38 g de base de l'exemple 5, 1,76 g de borhydrure de sodium et 3,4cm3 de pyridine dans 85 cm3 d'éthanol, dilue à l'eau en refroidissant au bain de glace, extrait à l'acétate d'éthyle, lave la phase organique à l'eau, à l'eau salée, sèche sur déshydratant, filtre, amène à sec, ajoute du pentane, filtre les cristaux, les lave à l'isopropanol, les sèche sous pression réduite et obtient 2,576 g du produit attendu F ≃ 140° C.

Exemple 7 : N-/3-/2-/3-(1 H-indol-4-yl) propyl/ phénoxy/ propyl/-2-méthyl-2-propanamine et son (E) butène dioate neutre

A 30 cm3 d'ammoniac à -78° C, on ajoute 3,38 g de produit obtenu comme à l'exemple 6 et 25 cm3 de tétrahydrofuranne, puis 3 g de sodium à -40° C, agite sous atmosphère inerte pendant 1 H 30, ajoute alors 24 g de chlorure d'ammonium à -40° C, laisse évaporer, dilue à l'eau, extrait à l'acétate d'éthyle, lave à l'eau, à l'eau salée, sèche sur déshydratant, filtre, amène à sec et obtient 2,86 g du produit attendu.

Formation du (E) butène dioate neutre

On ajoute 0,898 g d'acide fumarique dans 60 cm3 d'acétone chaud à une solution de 2,82 g de base ci-dessus dans 30 cm3 d'acétone, filtre, lave à l'isopropanol, sèche sous pression réduite, chauffe dans 100 cm3 d'éthanol, filtre, sèche à 80 ° C sous pression réduite et obtient 2,165 g du produit attendu F ≃ 230° C.

| Analyse | $C_{24}H_{32}N_2O, \frac{1}{2} (C_4H_4O_4)$ = 422,572 | | | | | |
|---|---|---|---|---|---|---|
| Calculé | C % | 73,90 | H % | 8,11 | N % | 6,63 |
| Trouvé | | 69,98 | | 7,55 | | 5,83 |

Exemple 8 : 1-/2-/2-/(1,1-diméthyléthyl) amino/ éthoxy/ phényl/3-/1 H-indol-4-yl 2-propèn-1-ol

On ajoute 1,1 cm3 de pyridine et 624 mg de borohydrure de sodium sous atmosphère inerte, à une solution de 1,5 g de base obtenue à l'exemple 1 dans 35 cm3 d'éthanol, agite pendant 15 mn à 70¤ C, ajoute de l'eau, refroidit dans un bain de glace, extrait à l'acétate d'éthyle, lave à l'eau, sèche sur déshydratant, amène à sec, purifie par chromatographie sur silice (éluant acétate d'éthyletriéthylamine 9-1) et obtient 1,32 g du produit attendu

Spectre UV (éthanol)

| | | | | |
|---|---|---|---|---|
| Max. | 221 nm | $E_1^1$ | 685 | ε = 24 968 |
| Infl. | 233 nm | $E_1^1$ | 533 | |
| Infl. | 276 nm | $E_1^1$ | 151 | |
| Infl. | 285 nm | $E_1^1$ | 186 | |
| Infl. | 292 nm | $E_1^1$ | 219 | |
| Max. | 308 nm | $E_1^1$ | 267 | ε = 9732 |

Exemple 9 : /N-/2-/2-/3-(1 H-indol-4-yl) propyl/ phénoxy/ éthyl/2-méthyl-2-propanamine/ et son (E9) butène dioate neutre

A 30 cm3 d'ammoniac à -78¤ C, on ajoute une solution de 3,936 g de produit de l'exemple 8 dans 25 cm3 de tétrahydrofuranne, porte le mélange à -40¤ C, ajoute 4,2 g de sodium en morceaux, poursuit l'agitation pendant 2 H sous atmosphère inerte, ajoute 36 g de chlorure d'ammonium à -40¤ C, laisse évaporer, dilue à l'eau en refroidissant dans un bain de glace, extrait à l'acétate d'éthyle, lave à l'eau, sèche sur déshydratant, filtre, amène à sec, lave à l'isopropanol, sèche sous pression réduite et obtient 2,43 g du produit attendu F = 114¤ C.

Formation du (E) butène dioate neutre

A une solution de 2,4 g de base ci-dessus, dans 90 cm3 d'éthanol on ajoute, sous agitation 0,795 g d'acide fumarique, concentre, amorce la cristallisation, abandonne 16 H à + 4¤C, filtre, sèche sous pression réduite, et obtient 1,67 g du produit attendu F = 190¤ C.

Analyse : $C_{23}H_{30}N_2O, \frac{1}{2} (C_4H_4O_4) = 408,545$

| | | | | | | |
|---|---|---|---|---|---|---|
| Calculé | C % | 73,50 | H % | 7,89 | N % | 6,86 |
| Trouvé | | 73,3 | | 7,9 | | 7,2 |

Exemple 10 : 1-/4-/2-/(1,1-diméthyléthyl) amino/ éthoxy/ phényl/3-(1H-indol-4-yl) 2-propèn-1-one (E) et son éthane dioate neutre

On ajoute sous agitation et atmosphère inerte 2,55 g d'indole-4-carboxaldéhyde et 4 cm3 de soude à 50 % à une solution de 3,5 g de 1-/4-/2 (1,1-diméthyléthyl) aminoéthoxy/ phényl/ éthanone dans 40 cm3 d'éthanol ; après 24 H d'agitation, on dilue à l'eau, extrait à l'acétate d'éthyle, lave à l'eau, réextrait la phase aqueuse avec un mélange chloroforme-méthanol 8-2, lave à l'eau, à l'eau salée, réunit les phases organiques, sèche sur déshydratant, amène à sec, purifie par chromatographie sur silice (éluant : acétate d'éthyle-triéthylamine 9-1) et obtient 4,3 g du produit attendu sous forme de base F = 162¤ C.

Formation de l'éthane dioate neutre

On ajoute à 80¤ C sous agitation une solution de 1,043 g d'acide oxalique dihydraté dans 20 cm3 de méthanol à une suspension de 3 g de base ci-dessus dans 40 cm3 d'éthanol et 55 cm3 de méthanol. On filtre, évapore le solvant sous pression réduite, reprend à chaud dans un mélange de 40 cm3 de méthanol, 100 cm3 d'éthanol et 100 cm3 d'eau, filtre à chaud, concentre et laisse cristalliser.

On filtre et sèche à 80¤ C sous pression réduite les cristaux obtenus, et obtient 1,1 g, du produit attendu F = 248¤ C.

Analyse : $C_{23}H_{26}N_2O_2, \frac{1}{2} C_2H_2O_4$ = 407,494

| | | | | | | |
|---|---|---|---|---|---|---|
| Calculé | C% | 70,74 | H% | 6,68 | N% | 6,87 |
| Trouvé | | 70,5 | | 6,8 | | 6,7 |

Préparation de la 1-/4-/2-(1,1-diméthyléthyl) aminoéthoxy/ phényl/éthanone.

Stade A : 1-/4-(2-chloréthoxy- phényl/ éthanone.

On porte au reflux pendant 18 H, sous atmosphère inerte, un mélange de 100 mg de 4-hydroxy acétophénone, 96 mg d'hydrogénosulfate de n-tétrabutyl ammonium, 1,5 cm3 de soude à 50%, 3 cm3 de benzène, 1,5 cm3 d'acétonitrile et 0,2 cm3 de 2-chloroéthyl paratoluène sulfonate. On décante, extrait au benzène, lave à l'eau, sèche sur déshydratant, amène à sec, purifie par chromatographie sur silice (éluant : dichlorométhane) et obtient 127 mg du produit attendu F ≃ 62° C.

Stade B : 1-/4-/2-(1,1-diméthyléthyl amino éthoxy/ phényl/éthanone.

On chauffe à 120°C pendant 30 H un mélange de 6,5 g de produit du stade A dans 30 cm3 de tert butylamine, dilue à l'eau et à l'acétate d'éthyle, décante, lave à l'eau, sèche sur déshydratant, filtre, évapore le solvant, purifie par chromatographie sur silice (éluant : acétate d'éthyle-triéthylamine 9-1) et obtient 7,264 g du produit attendu.

Exemple 11 : 1-/2-/2-/(1,1-diméthyléthyl) amino/ propoxy/phényl/3-/1H-indol-4-yl/ 1-propanone et son chlorhydrate

En opérant selon un procédé analogue à celui décrit à l'exemple 2, mais à partir de 3,616 g 1-/2-/2-/(1,diméthyléthyl) amino/ propoxy/phényl/3 /1 H-indol-4-yl/1-propanone de l'exemple 5, on obtient 2,98 g de produit attendu F ≃ 64°C.

Formation du chlorhydrate.

On dissout 2 g de produit obtenu ci-dessus dans 30 cm3 d'isopropanol chaud, ajoute de l'acétate d'éthyle chlorhydrique jusqu'à pH acide, glace, filtre, sèche à 80°C sous pression réduite, recristallise dans l'isopropanol et obtient 1,22 g du produit attendu. F≃162°C.

Spectre UV (éthanol)

| | | | | |
|---|---|---|---|---|
| Max. | 215 nm | $E_1^1$ | 1278 | ε = 53 000 |
| Max. | 251 nm | $E_1^1$ | 257 | ε = 10 700 |
| Max. | 270 nm | $E_1^1$ | 198 | ε = 8 200 |
| Max. | 276 nm | $E_1^1$ | 192 | ε = 7 950 |
| Infl. | 282 nm | $E_1^1$ | 188 | |
| Max. | 288 nm | $E_1^1$ | 167 | ε = 6950 |
| Infl. | 301 nm | $E_1^1$ | 92 | |

Exemple 12 : α-/2-/3-/(1,1-diméthyléthyl) amino/ propoxy/ phényl/1H-indol-4-propanol (E) butène dioate neutre.

En opérant selon un procédé analogue à celui décrit à l'exemple 3, mais à partir de 2,937 g de base obtenue à l'exemple 11, on obtient 2,86 g de produit attendu. F≃240°C après recristallisation dans le méthanol.

<u>Analyse</u> : $C_{24}H_{32}N_2O_2, \frac{1}{2}C_4H_4O_4$ = 438,571.

| | | | |
|---|---|---|---|
| Calculé : | C % 71,21 | H % 7,81 | N % 6,39 |
| Trouvé : | 71,1 | 7,9 | 6,4. |

<u>Exemple 13 : N-/2-/2-/3-(1H-indol-4-yl) propyl/phénoxy/éthyl/ 2-méthyl-2-propanamine.</u>

En opérant selon un procédé analogue à celui décrit à l'exemple 4, mais à partir de la base obtenu à l'exemple 1 et en laissant réagir pendant 6 heures, on obtient le produit attendu. F ≃ 112°C.
Rf = 0,39 (acétate d'éthyle - triéthylamine 9/1).

<u>Exemple 14: 1-/2-/2-(diméthylamino)éthoxy/phényl/3-(1H-indol-4-yl)1-propanone.</u>

<u>Stade A</u> : 3-(1H-indol-4-yl) 1-(2-hydroxyphényl) 2-propèn-1-one.

On ajoute à 30°C sous agitation et atmosphère inerte 0,5 cm3 de potasse à 38 % à un mélange de 0,132 g d'indol-4-carboxaldéhyde, de 0,1 cm3 de 2-hydroxyacétophénone et de 0,189 g de chlorure de triéthylbenzyl ammonium dans 2 cm3 d'éthanol. Après 23 heures d'agitation à 30°C, on dilue à l'eau, extrait à l'acétate d'éthyle, lave à l'eau, sèche sur déshydratant, amène à sec, purifie par chromatographie sur silice (éluant : chlorure de méthylène) et obtient 159 mg du produit attendu. F ≃ 164°C.

<u>Stade B</u> :1-/2-hydroxyphényl/3-1H-indol-4-yl/1-propanone.

On hydrogène jusqu'à fin d'absorption 200 mg de produit du stade A dans 10 cm3 de méthanol en présence de charbon palladié, filtre, évapore le méthanol, purifie par chromatographie sur silice (éluant : cyclohexane - dichlorométhane - triéthylamine 6-3-1) et obtient 143 mg du produit attendu.
F≃143°C.

Spectre UV (éthanol)

| | | | | |
|---|---|---|---|---|
| Max. | 214 nm | $E_1^1$ | 2080 | ε = 55 200 |
| Max. | 254 nm | $E_1^1$ | 555 | ε = 14 700 |
| Infl. | 275 nm | $E_1^1$ | 295 | |
| Infl. | 286 nm | $E_1^1$ | 220 | |
| Max. | 323 nm | $E_1^1$ | 117 | ε = 4700 |

<u>Stade C</u> : 1-/2-/2-(diméthylamino)éthoxy/phényl/3-(1H-indol-4-yl)1-propanone.

On place sous agitation et atmosphère inerte, 2,7 g de produit du Stade B dans 50 cm3 de benzène et 25 cm3 d'acétonitrile avec 345,5 mg d'hydrogénosulfate de tétrabutylammonium, ajoute 39 cm3 de solution aqueuse de soude à 50 %, chauffe à 80°C, ajoute 1,47 g de chlorhydrate de chlorure de diméthylamino éthyle puis, toutes les heures pendant 3 heures, 0,738 g du chlorhydrate. On dilue à l'eau, à l'acétate d'éthyle, décante, réextrait à l'acétate d'éthyle la phase aqueuse, lave à l'eau les phases organiques réunies, sèche sur déshydratant, filtre, amène à sec, purifie par chromatographie sur silice (éluant : chloroforme - acétone - triéthylamine 6-3-1) et obtient 2,81 g du produit attendu.
F≃64°C.

| Spectre UV (éthanol) | | | | |
|---|---|---|---|---|
| Max. | 216 nm | $E_1^1$ | 1589 | ε = 53 500 |
| Max. | 249 nm | $E_1^1$ | 286 | ε = 9600 |
| Max. | 270 nm | $E_1^1$ | 250 | ε = 8400 |
| Max. | 278 nm | $E_1^1$ | 247 | ε = 8300 |
| Max. | 289 nm | $E_1^1$ | 213 | ε = 7200 |
| Infl. | 301 nm | $E_1^1$ | 109 | |

Exemple 15: l' -/2-/2-(diméthylamino) éthoxy/phényl/1H-indol-4-propanol et son tartrate acide.

On agite sous atmosphère inerte une solution de 2,43 g de produit de l'exemple 14 dans 60 cm3 de méthanol avec 0,820 g de borohydrure de sodium pendant 10 minutes, ajoute de l'eau en refroidissant dans un bain de glace, extrait à l'acétate d'éthyle, lave à l'eau, sèche sur déshydratant, filtre, évapore à sec et obtient 2,42 g du produit attendu.

| Spectre UV (éthanol) | | | | |
|---|---|---|---|---|
| Max. | 218 nm | $E_1^1$ | 1308 | ε = 44 300 |
| Max. | 270 nm | $E_1^1$ | 279 | ε = 9450 |
| Infl. | 276 nm | $E_1^1$ | 260 | |
| Max. | 288 nm | $E_1^1$ | 139 | ε = 4700 |

Formation du tartrate.

On ajoute 0,795 g d'acide DL tartrique dans 30 cm3 de méthanol chaud à une solution de 1,8 g d'α-/2-/2-(diméthylamino)éthoxy/phényl/1H-indol-4-propanol dans 30 cm3 de méthanol, amorce la cristallisation, refroidit, essore,puis recristallise dans le méthanol et obtient 0,65 g du produit attendu. F≈170¤C.

| Spectre UV (éthanol) | | | | |
|---|---|---|---|---|
| Max. | 217 nm | $E_1^1$ | 900 | ε = 44 000 |
| Max. | 269 nm | $E_1^1$ | 187 | ε = 9100 |
| Infl. | 274 nm | $E_1^1$ | 175 | |
| Infl. | 281 nm | $E_1^1$ | 138 | |
| Max. | 288 nm | $E_1^1$ | 97 | ε = 4750 |

Exemple 16: le 1-/2-/2-(diméthylamino)éthoxy/phényl/3-(1H-indol-4-yl) 2-propèn-1-one et son oxalate acide.

On place sous agitation et atmosphère inerte, 3 g de produit du Stade A de l'exemple 14 dans 50 cm3 de benzène et 25 cm3 d'acétonitrile avec 387 mg d'hydrogénosulfate de tétrabutylammonium, ajoute 45 cm3 de soude à 50 %, chaufe à 50¤C, ajoute 1,64 g de chlorhydrate de chlorure de diméthylaminoéthyle, puis, toutes les heures (soit 3 heures), 0,820 g dudit chlorhydrate. Après 4 heures, on dilue à l'eau, à l'acétate d'éthyle, décante, extrait à l'acétate d'éthyle, lave à l'eau les phases organiques réunies, sèche sur déshydratant, filtre, amène à sec, purifie par chromatographie sur silice (éluant : $CHCl_3$ - acétone - triéthylamine 6-3-1) et obtient 3,7 g du produit attendu.

Spectre UV (éthanol)

| | | | | |
|---|---|---|---|---|
| Max. | 216 nm | $E_1^1$ | 870 | ε = 29 100 |
| Max. | 266 nm | $E_1^1$ | 353 | ε = 11 800 |
| Infl. | 344 nm | $E_1^1$ | 246 | |
| Max. | 387 nm | $E_1^1$ | 380 | ε = 12 700 |

Formation de l'oxalate acide.

On ajoute 0,819 g d'acide oxalique dihydraté en solution dans 20 cm3 de méthanol, à une solution de 2,18 g de base obtenu ci-dessus dans 40 cm3 de méthanol, ajoute 30 cm3 d'isopropanol, concentre, amorce la cristallisation, refroidit, filtre, sèche à 80°C sous pression réduite et obtient 2 g du produit attendu. F≃174°C.

Spectre UV (éthanol)

| | | | | |
|---|---|---|---|---|
| Max. | 264 nm | $E_1^1$ | 330 | ε = 14 007 |
| Infl. | 358 nm | $E_1^1$ | 250 | |
| Max. | 390 nm | $E_1^1$ | 354 | ε = 15 025 |

Exemple 17: la N,N-(diméthyl 2-/2-/3-(1H-indol-4-yl)propyl/ phénoxy/éthanamine et son tartrate acide.

On ajoute 2,245 g de la base obtenue à l'exemple 15, en solution dans 25 cm3 de tétrahydrofuranne, à 25 cm3 d'ammoniac condensé à - 78°C, ajoute 2 g de morceaux de sodium à -40°C, agite pendant une heure 15 à -40°C environ, ajoute 16 g de chlorure d'ammonium à -40°C, laisse évaporer lentement l'ammoniac à température ambiante, ajoute lentement de l'eau, extrait à l'acétate d'éthyle, lave à l'eau, sèche sur déshydratant, filtre, amène à sec, purifie par chromatographie sur silice (éluant : acétate d'éthyle - triéthylamine 9-1) et obtient 1,9 g du produit attendu.
F≃ 78°C.

Spectre UV (éthanol)

| | | | | |
|---|---|---|---|---|
| Max. | 220 nm | $E_1^1$ | 1687 | ε = 54 400 |
| Max. | 271 nm | $E_1^1$ | 375 | ε = 12 000 |
| Max. | 279 nm | $E_1^1$ | 350 | ε = 11 300 |
| Max. | 289 nm | $E_1^1$ | 192 | ε = 6200 |

Formation du tartrate acide.

On ajoute une solution de 0,780 g d'acide DL tartrique dans 30 cm3 d'éthanol chaud à une solution de 1,68 g de base obtenue ci-dessus en solution dans 40 cm3 d'éthanol chaud. Le tartrate cristallise ; on filtre, recristallise dans le méthanol et obtient 2,153 g du produit attendu. F≃190°C.

Spectre UV (éthanol)

| | | | | |
|---|---|---|---|---|
| Max. | 219 nm | $E_1^1$ | 1060 | ε = 50 100 |
| Infl. | 267 nm | $E_1^1$ | 211 | |
| Max. | 270 nm | $E_1^1$ | 225 | ε = 10 600 |
| Max. | 277 nm | $E_1^1$ | 211 | ε = 10 000 |
| Max. | 289 nm | $E_1^1$ | 119 | ε = 5600 |

Exemple 18: 1-/2-/2-(1-pipéridinyl)éthoxy/phényl/3-(1H-indol-4-yl) 1-propanone.

En opérant selon un procédé analogue à celui décrit à l'exemple 14, Stade C, mais à partir de chlorhydrate de 2-pipéridino 1-chloro éthane, on obtient le produit attendu.
F = 78°C

Spectre UV (éthanol)

| | | | | |
|---|---|---|---|---|
| Max. | 215 nm | $E_1^1$ | 1454 | ε = 54 700 |
| Max. | 248 nm | $E_1^1$ | 264 | ε = 9900 |
| Max. | 268 nm | $E_1^1$ | 225 | ε = 8500 |
| Max. | 278 nm | $E_1^1$ | 221 | ε = 8300 |
| Infl. | 280 nm | $E_1^1$ | 217 | |
| Max. | 288 nm | $E_1^1$ | 190 | ε =7150 |
| Infl. | 301 nm | $E_1^1$ | 101 | |

Exemple 19 :1-/2-/2 (1-pipéridinyl)éthoxy/phényl/3-(1H-indol-4-yl) 2-propèn-1-one et sone oxalate acide.

En opérant selon un procédé analogue à celui décrit à l'exemple 16 mais à partir de chlorhydrate de 2-pipéridino 1-chloroéthane, on obtient le produit attendu et prépare son oxalate acide qui fond à 174°C

Spectre UV (éthanol)

| | | | | |
|---|---|---|---|---|
| Max. | 265 nm | $E_1^1$ | 297 | ε = 13 800 |
| Infl. | 345 nm | $E_1^1$ | 195 | |
| Max. | 390 nm | $E_1^1$ | 319 | ε = 14 800 |

Exemple 20: α-/2-/2-(1-pipéridinyl)éthoxy/phényl/1H-indol-4-propanol et son fumarate neutre.

En opérant selon un procédé analogue à celui décrit à l'exemple 15, mais à partir du produit de l'exemple 18, on obtient le produit attendu et prépare le fumarate neutre qui fond à 190°C.

Spectre UV (éthanol)

| | | | | |
|---|---|---|---|---|
| Max. | 218 nm | $E_1^1$ | 1190 | ε = 52 000 |
| Max. | 270 nm | $E_1^1$ | 231 | ε = 10 100 |
| Infl. | 276 nm | $E_1^1$ | 215 | |
| Infl. | 281 nm | $E_1^1$ | 180 | |
| Infl. | 288 nm | $E_1^1$ | 117 | |

Exemple 21: 4-/3-/2-/2-(1-pipéridinyl)éthoxy/phényl/propyl/1H-indole et son tartrate acide.

En opérant selon un procédé analogue à celui décrit à l'exemple 17, mais à partir de produit de l'exemple 20, on obtient le produit attendu et prépare le tartrate acide qui fond à 125°C.

Spectre UV (éthanol)

| | | | | |
|---|---|---|---|---|
| Max. | 217 nm | $E_1^1$ | 818 | ε = 41 900 |
| Max. | 270 nm | $E_1^1$ | 975 | ε = 9000 |
| Max. | 276 nm | $E_1^1$ | 166 | ε = 8500 |
| Infl. | 282 nm | $E_1^1$ | 125 | |
| Max. | 288 nm | $E_1^1$ | 93 | ε = 4750 |

Exemple 22:22:1-/2-/2-/bis(1-méthyléthyl)amino/éthoxy/phényl/3-1H-indol-4-yl) 1-propanone.

En opérant selon un procédé analogue à celui décrit à l'exemple 14, Stade C mais à partir de chlorhydrate de chlorure de diisopropyle amino éthyle, on obtient le produit attendu.

| Spectre UV | | | | |
|---|---|---|---|---|
| Max. | 214 nm | $E_1^1$ | 1292 | ε = 50 700 |
| Max. | 249 nm | $E_1^1$ | 239 | ε = 9400 |
| Max. | 270 nm | $E_1^1$ | 201 | ε = 7900 |
| Max. | 279 nm | $E_1^1$ | 199 | ε = 7800 |
| Max. | 290 nm | $E_1^1$ | 170 | ε = 6700 |
| Infl. | 301 nm | $E_1^1$ | 88 | ε = 3450 |

Exemple 23 : 1-/2-/2-/bis(1-méthyléthyl)amino/éthoxy/phényl/3-(1H-indol-4-yl)2-propèn-1-one et son oxalate acide.

En opérant selon un procédé analogue à celui décrit à l'exemple 16 mais à partir de chlorhydrate de chlorure de diisopropylamino éthyle, on obtient le produit attendu et prépare l'oxalate acide.

| Spectre UV | | | | |
|---|---|---|---|---|
| Infl. | 213 nm | $E_1^1$ | 685 | |
| Max. | 266–267 nm | $E_1^1$ | 272 | ε = 13 000 |
| Infl. | 342 nm | $E_1^1$ | 159 | |
| Max. | 393 nm | $E_1^1$ | 293 | ε = 14 000 |

Exemple 24 : α-/2-/2-/bis(1-méthyléthyl)amino/éthoxy/phényl/1H-indol-4-propanolet son benzoate.

En opérant selon un procédé analogue à celui décrit à l'exemple 15, mais à partir du produit de l'exemple 22, on obtient le produit attendu.
F≈110°C (benzoate).

| Spectre UV (éthanol) | | | | |
|---|---|---|---|---|
| Max. | 220 nm | $E_1^1$ | 1136 | ε = 58 700 |
| Infl. | 267 nm | $E_1^1$ | 205 | |
| Max. | 270 nm | $E_1^1$ | 216 | ε = 11 200 |
| Infl. | 276 nm | $E_1^1$ | 200 | |
| Max. | 289 nm | $E_1^1$ | 104 | ε = 5400 |

Exemple 25 : N-/2-/2-/3-(1H-indol-4-yl)propyl/phénoxy/éthyl/N-(1-méthyl éthyl) 2-propanamine et son oxalate acide.

En opérant selon un procédé analogue à celui décrit à l'exemple 17, mais à partir du produit de l'exemple 24, on obtient le produit attendu.
F≈108-112°C. (oxalate acide).

Spectre UV (éthanol)

| | | | | |
|---|---|---|---|---|
| Max. | 220 nm | $E_1^1$ | 938 | ε = 44 000 |
| Max. | 270 nm | $E_1^1$ | 198 | ε = 9300 |
| Max. | 277 nm | $E_1^1$ | 187 | ε = 8800 |
| Max. | 289 nm | $E_1^1$ | 106 | ε = 5000 |

Exemple 26 : 1,3-dihydro 4-/3-/2-/2-(1,1-diméthyléthyl) amino/ éthoxy/phényl/ propyl/ 2H-indol-2-one et son fumarate neutre.

Stade A : N-/2-/2-/3-(3-chloro 1H-indol-4-yl) propyl/ phénoxy/éthyl/ 2-méthyl 2-propanamine.

On porte sous agitation et atmosphère inerte 4,74 g de produit obtenu à l'exemple 9 dans 94 cm3 d'acide acétique avec 2,17 g de N-chlorosuccinimide pendant 40 minutes, dilue à l'eau, glace, alcalinise à l'aide de soude à 32 %, extrait à l'acétate d'éthyle, lave à l'eau, sèche sur déshydratant filtre, amène à sec, purifie par chromatographie sur silice (éluant : acétate d'éthyle - triéthylamine 9-1) et obtient 3,76 g de produit attendu.

Stade B : 1,3-dihydro 4-/3-/2-/2-/(1,1-diméthyléthyl)amino/ éthoxy/phényl/ propyl 2H-indol-2-one et son fumarate neutre.

On agite pendant 19 heures sous amosphère inerte 3,76 g de produit du stade A dans 94 cm3 de solution aqueuse d'acide chlorhydrique N et 94 cm3 d'éthanol à 96 %, dilue à l'eau, glace, alcalinise à la soude à 32 %, extrait à l'acétate d'éthyle, lave à l'eau, sèche sur déshydratant, filtre, amène à sec, purifie par chromatographie sur silice (éluant : acétate d'éthyle - triéthylamine 9-1) et obtient 2,18 g du produit attendu.

Formation du fumarate neutre.

On dissout la base ci-dessus dans 10 cm3 d'isopropanol chaud, ajoute 0,690 g d'acide fumarique, glace, filtre, sèche, recristallise dans un mélange méthanol - isopropanol (1-7), 1,77 g du produit attendu. F≈193°C

Spectre UV (éthanol)

| | | | | |
|---|---|---|---|---|
| Infl. | 216 nm | $E_1^1$ | 769 | |
| Infl. | 247 nm | $E_1^1$ | 219 | |
| Max. | 250 nm | $E_1^1$ | 255 | ε = 9600 |
| Infl. | 259 nm | $E_1^1$ | 174 | |
| Infl. | 269 nm | $E_1^1$ | 93 | |
| Max. | 276 nm | $E_1^1$ | 79 | ε = 3350 |
| Infl. | 288 nm | $E_1^1$ | 30 | |

Exemple 27: 1,3-dihydro 40/3-/2-/3-/(1,1-diméthyléthyl) amino/ propoxy/phényl/ propyl/ 2H-indol-2-one et son fumarate neutre.

En opérant selon un procédé analogue à celui décrit à l'exemple 26, on obtient le produit attendu. F≈223°C (fumarate neutre).

Préparation du 2-/3-(1H-indol-4-yl) propyl/phénol.

On ajoute lentement sous agitation 22,6 cm3 d'hydrate d'hydrazine, puis 11,6 g de produit du Stade B de l'exemple 14 à 50 cm3 de diéthylène glycol, ajoute 20 cm3 de lessive de potasse à 38 %, porte à 140°C pendant 30 minutes sous atmosphère inerte, distille l'eau et l'hydrate d'hydrazine en excès à 210°C, agite encore pendant 2 heures, refroidit au bain de glace, ajoute de l'eau, extrait à l'acétate d'éthyle, lave à l'eau, sèche au déshydratant, filtre, amène à sec le filtrat, purifie le résidu par chromatographie sur silice (éluant : chlorure de méthylène) et obtient 8,613 g du produit attendu. F≈89°C.

| Spectre UV (éthanol) | | | | |
|---|---|---|---|---|
| Max. | 219 nm | $E_1^1$ | 1705 | ε = 42 900 |
| Infl. | 260 nm | $E_1^1$ | 313 | |
| Max. | 273 nm | $E_1^1$ | 380 | ε = 9550 |
| Infl. | 278 nm | $E_1^1$ | 375 | |
| Max. | 289 nm | $E_1^1$ | 225 | ε = 5650 |
| Infl. | 310 nm | | | |
| Infl. | 334 nm | | | |

Exemple 28 :

On a préparé des comprimés répondant à la formule :
- fumarate neutre de N-/2-/2-3-(1H-indol-4-yl) propyl/ phénoxy/éthyl/ 2-méthyl-2-propanamine ............... 100 mg,
- excipient q.s. pour un comprimé terminé à........... 150 mg.
(Détail de l'excipient : lactose, amidon, talc stéarate de magnésium).

Exemple 29 :

On a préparé des comprimés répondant à la formule :
- chlorhydrate de 1-/2-/2-/(1,1-diméthyléthyl) amino/ éthoxy/phényl/ 3-/1H-indol-4-yl 1-propanone................ 100 mg.
- excipient q.s. pour un comprimé terminé à........... 150 mg.
(Détail de l'excipient : lactose, amidon, talc, stéarate de magnésium).

Exemple 30 :

On a préparé des comprimés répondant à la formule :
- oxalate acide de la N-/2-/2-/3-(1H-indol-4-yl) propyl/ phénoxy/éthyl/ N-(1-méthyléthyl) 2-propanamine.................... 100 mg.
- Excipient q.s.pour un comprimé terminé à ........... 150 mg.
(Détail de l'excipient : lactose, amidon, talc, stéarate de magnésium).

Exemple 31 :

On a préparé des comprimés répondant à la formule :
- chlorhydrate du 1-/2-/3-/(1,1-diméthyl éthyl) amino/ propoxy/phényl/ 3-(1H-indol-4-yl) 1-propanone...................... 100 mg.
- Excipient q.s.pour un comprimé terminé à............. 150 mg.
(Détail de l'excipient : lactose, amidon, talc, stéarate de magnésium).

Etude pharmacologique.

1) Action antiarythmique chez le rat.

On trachéotomise des rats mâles pesant 300-350 g anesthésiés par voie intrapéritonéale à l'aide de 1,20 g/kg d'uréthane et les soumet à une respiration artificielle (40-50 insufflations de 3 ml/minute).

On implante des aiguilles en sous cutané de manière à enregistrer l'électrocardiogramme des rats sur le signal en dérivation DII.

On administre les produits à tester par voie intraveineuse ou par voie orale.

Cinq minutes après l'administration du produit, on perfuse la veine jugulaire des rats avec 10 µg/mn sous 0,2 ml d'une solution d'aconitine et on note le temps d'apparition des troubles du rythme cardiaque.

Les résultats sont exprimés en pourcentage d'allongement du temps d'apparition des troubles du rythme cardiaque par rapport aux témoins et en fonction de la dose du produit testé.

Les résultats figurant sur le tableau ci-après montrent que les produits de la présente demande sont doués de remarquables propriétés antiarythmiques.

| Produit de l'exemple | voie | Dose | Pourcentage d'allongement du temps |
|---|---|---|---|
| 1 | IV | 0,5 mg/kg | 26 |
| | PO | 10 mg/kg | 41 |
| 2 | IV | 2,5 mg/kg | 40 |
| | PO | 25 mg/kg | 32 |
| 3 | IV | 0,05 mg/kg | 36 |
| | PO | 5 mg/kg | 38 |
| 5 | IV | 2,5 mg/kg | 56 |
| | PO | 2,5 mg/kg | 41 |
| 9 | IV | 2,5 mg/kg | 49 |
| | PO | 25 mg/kg | 40 |
| 11 | IV | 1 mg/kg | 42 |
| | PO | 25 mg/kg | 66 |
| 20 | IV | 2,5 mg/kg | 50 |
| 24 | IV | 0,25 mg/kg | 25 |
| | PO | 5 mg/kg | 46,5 |
| 26 | IV | 1 mg/kg | 25,5 |
| 28 | IV | 0,25 mg/kg | 24 |

(2) Test d'activité anticalcique in vitro.

Des artères caudales de rat découpées en spirale sont reliées à des capteurs de tension et sont maintenues dans des cuves de 25 ml de tampon Krebs-bicarbonate de sodium (NaCl : 120,8 mM, KCl ; 5,9 mM, $MgCl_2$ : 1,2 mM, $NaH_2PO_4$ : 1,2 mM, $NaHCO_3$ : 15,5 mM, glucose : 12,6 mM) à 37¤C gazées avec un mélange : $O_2$ : 95 % - $CO_2$ : 5 %.

Les préparations sont dépolarisées par une solution tampon à concentration 100 mM en ions $K^+$ (NaCl : 26,7 mM, KCl : 100 mM, $MgCl_2$ 1,2 mM, $NaH_2PO_4$ : 1,2 mM, $NaHCO_3$ : 15,5 mM, glucose : 12,6 mM).

On ajoute, sous un volume de 250 µl, du chlorure de calcium à la concentration de 2,5 mM et on enregistre les contractions des artères ainsi induites. On reèpète l'opération toutes les 15 minutes, chaque contraction est suivie de 2 lavages par une solution tampon dépourvue de calcium.

Lorsqu'une réponse stable est obtenue, l'opération est renouvelée en présence de concentrations croissantes du produit à tester. Le temps de contact pour chaque concentration est de 15 minutes.

Les contractions des artères dépendent de l'entrée des ions $Ca^{2+}$ dans les cellules musculaires lisses et sont provoquées par la dépolarisation des cellules par les ions $K^+$ et par l'action de la noradrénaline libérée au niveau présynaptique. Afin de supprimer l'action vasoconstrictrice de la noradrénaline, les essais sont réalisés en présence du phéntolamine $10^{-5M}$, antagoniste alpha-adrénergique.

Les résultats sont exprimés en $CI_{50}$ (concentration inhibitrice 50) concentration du produit testé qui inhibe de 50 % la contraction due aux ions $K^+$.

On constate d'après le résultats figurant sur le tableau ci-après que les produits de la présente demande possèdent une forte activité anticalcique.

| Produit de l'exemple | CI 50 en µM |
|---|---|
| 1 | 1,9 |
| 2 | 0,8 |
| 3 | 1,9 |
| 5 | 1,4 |
| 7 | 0,9 |
| 9 | 0,8 |
| 11 | 0,26 |
| 17 | 1,2 |
| 19 | 1,5 |
| 20 | 0,71 |
| 21 | 0,52 |
| 24 | 1,3 |
| 25 | 0,23 |

3) Etude de la toxicité aigue.

On a évalué les doses léthales $DL_O$ des différents composés testés après administration par voie orale chez la souris.

On appelle $DL_O$ la dose maximale ne provoquant aucune mortalité en 8 jours.

Les résultats obtenus sont les suivants :

| Produit de l'exemple | $DL_0$ en mg/kg |
|---|---|
| 1 | >400 |
| 2 | 200 |
| 3 | 10 |
| 5 | 200 |
| 7 | 200 |
| 9 | 200 |
| 11 | 200 |
| 15 | 100 |
| 16 | 80 |
| 17 | >400 |
| 19 | >200 |
| 20 | 80 |
| 21 | 200 |
| 23 | 200 |
| 24 | 40 |
| 25 | 200 |
| 26 | 100 |

**Revendications**

1. Les dérivés du 4-phénylpropyl-indole ainsi que de leurs sels d'addition avec les acides minéraux ou organiques, caractérisés en ce qu'ils répondent à la formule générale I:

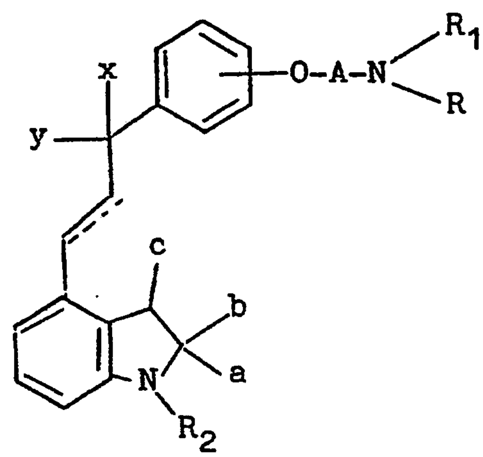

I

dans laquelle R et $R_1$ représentent, indépendamment l'un de l'autre, un atome d'hydrogène, un radical alkyle linéaire renfermant de 1 à 5 atomes de carbone, un radical alkyle ramifié renfermant de 3 à 5 atomes de carbone, un radical cycloalkyle renfermant de 3 à 7 atomes de carbone, un radical cycloalkylalkyle renfermant de 4 à 7 atomes de carbone, ou un radical aralkyle renfermant de 7 à 12 atomes de carbone, ou un radical aralkyle rentermant de 7 à 12 atomes de carbone substitué, choisi parmi les radicaux benzyle ou phénétyle substitués par 1, 2 ou 3 radicaux choisis dans le groupe constitué par les halogènes et les radicaux méthyle, éthyle, méthoxy, éthoxy, trifluorométhyle, méthylthio, amino et nitro, ou R et $R_1$ forment ensemble avec l'atome d'azote un cycle pyrrolidino, pipéridino, morpholino, pipérazinyle, méthyl pipérazinyle, éthyl pipérazinyle, propyl pipérazinyle a représente ensemble avec b une fonction oxo, ou représente ensemble avec c une liaison carbone-carbone, b représente un atome d'hydrogène, ou ensemble avec a une fonction oxo, c représente un atome d'hydrogène, ou ensemble avec a représente une liaison carbone-carbone, le pointillé représente la présence éventuelle d'une liaison carbone-carbone. A représente une chaîne $-(CH_2)_n-$ dans laquelle n peut prendre les valeurs 2, 3, 4 ou 5, $R_2$ représente un atome d'hydrogène, un radical alkyle linéaire renfermant de 1 à 5 atomes de carbone ou un radical alkyle ramifié renfermant de 3 à 5 atomes de carbone, x représente un radical hydroxyle, ou un atome d'hydrogène, ou ensemble avec y, une fonction oxo et y représente un atome d'hydrogène ou ensemble avec x, une fonction oxo.

2. Les dérivés du 4-phénylpropyl-indole tels que définis par la formule I de la revendication 1 ainsi leurs sels d'addition avec les acides minéraux ou organiques, caractérisés en ce que a et c représentent ensemble une liaison carbone-carbone.

3) Les dérivés du 4-phénylpropyl-indole selon la revendication 2, ainsi que leurs sels d'addition avec les acides minéraux ou organiques, caractérisés en ce que $R_1$ et $R_2$ représentent un atome d'hydrogène.

4) Le dérivé de formule I, selon la revendication I dont le nom suit : la N-/2-/2-/3-(1H-indol-4-yl) propyl/phénoxy/ éthyl/2-méthyl-2-propanamine,
ainsi que ses sels d'addition avec les acides minéraux ou organiques.

5) Le 1-/2-/3-/1,1-diméthyléthyl) amino/ propoxy/ phényl/3-(1H-indol-4-yl) 1-propanone ainsi que ses sels d'addition avec les acides minéraux ou organiques.

6) La N-/2-/2-/3-(1H-indol-4-yl) propyl/ phénoxy/ éthyl/ N-(1-méthyl éthyl) 2-propanamine ainsi que ses sels d'addition avec les acides minéraux ou organiques.

7) Procédé de préparation des dérivés du 4-phényl propyl-indole tels que définis par la formule I de la revendication 1 ainsi que de leurs sels, caractérisé en ce que l'on fait réagir un dérivé de formule II :

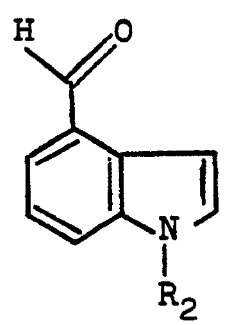

II

dans laquelle $R_2$ a la signification déjà indiquée avec un dérivé de formule III :

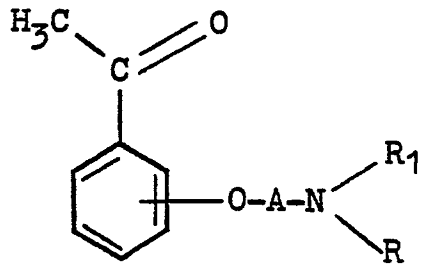

III

dans laquelle R et $R_1$ ont la signification déjà indiquée, pour obtenir un produit de formule $I_A$ :

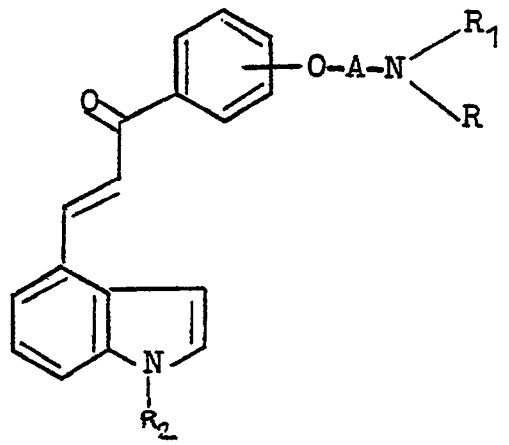

$I_A$

dans laquelle R, $R_1$ et $R_2$ ont la signification déjà indiquée, que soit l'on hydrogène par action d'hydrogène gazeux en présence d'un catalyseur :aga base de platine ou de palladium dans un solvant tel qu'un alcanol renfermant de 1 à 5 atomes de carbone, ou par action d'hydrogène gazeux en présence de Nickel de Raney dans un solvant tel que l'acétate d'éthyle, ou par action pendant moins de 3 heures, de sodium dans l'ammoniac, dans un solvant tel que le tétrahydrofuranne, pour obtenir un produit de formule $I_B$ :

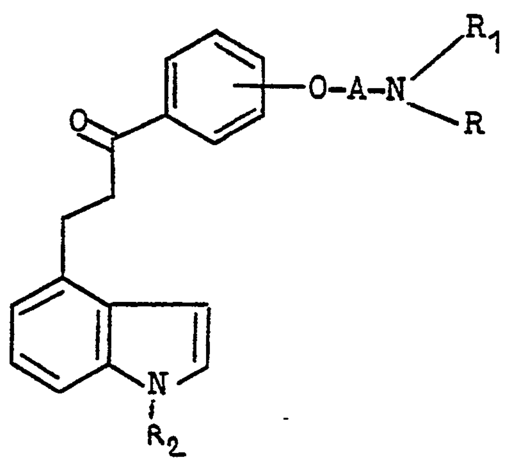

$I_B$

dans laquelle A, R, $R_1$ et $R_2$ ont la signification déjà indiquée, que ou bien l'on isole et, si désiré, salifie, ou bien l'on réduit à l'aide d'un borohydrure ou cyanoborohydrure alcalin pour obtenir un produit de formule $I_C$ :

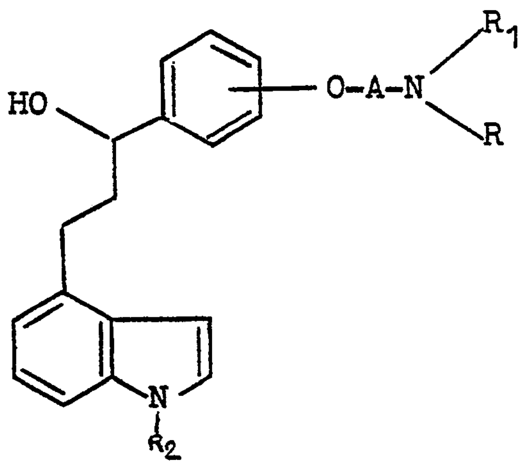

$I_C$

dans laquelle A, R, $R_1$ et $R_2$ ont la signification déjà indiquée, que ou bien l'on isole et, si désiré, salifie, ou bien l'on réduit à l'aide de sodium dans l'ammoniac pour obtenir un produit de formule $I_D$:

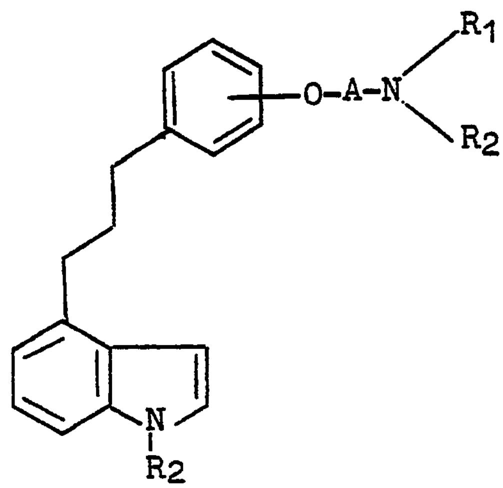

$I_D$

dans laquelle A, R, $R_1$ et $R_2$ ont la signification déjà indiquée, que l'on isole et, si désiré salifie, soit l'on fait réagir ledit produit de formule $I_A$ avec le complexe formé entre un borohydrure alcalin et la pyridine, pour obtenir un produit de formule $I_E$ :

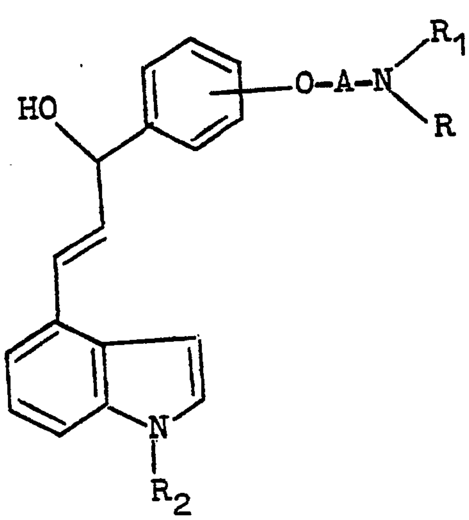

$I_E$

dans laquelle A, R, $R_1$ et $R_2$ ont la signification déjà indiquée, que ou bien l'on isole, ou bien l'on réduit à

l'aide de sodium dans l'ammoniac pour obtenir un produit de formule $I_D$ que l'on isole et, si désiré, salifie, soit l'on réduit ledit produit de formul $I_A$ à L'aide de sodium dans l'ammoniac pour un produit de formule $I_D$ que l'on isole et, si désiré salifie, puis soumet si désiré, lesdits produits de formule $I_A$, $I_B$, $I_C$, $I_D$ et $I_E$ à l'action d'un agent d'halogénation pour obtenir un produit de formule IV :

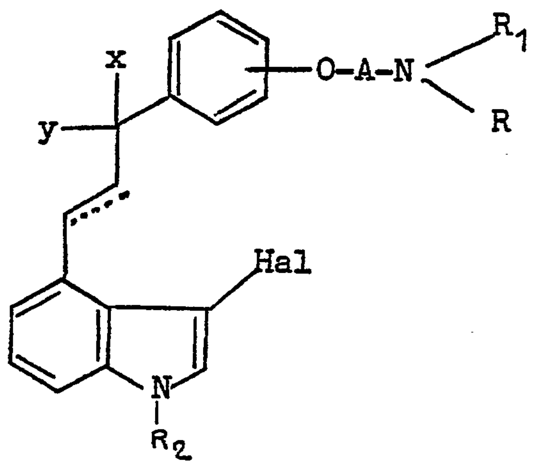

IV

dans laquelle Hal représente un atome de brome ou de chlore, et x, y, A, R, $R_1$, $R_2$ et le pointillé ont la signification déjà indiquée, que l'on soumet à une hydrolyse pour obtenir un produit de formule $I_F$ :

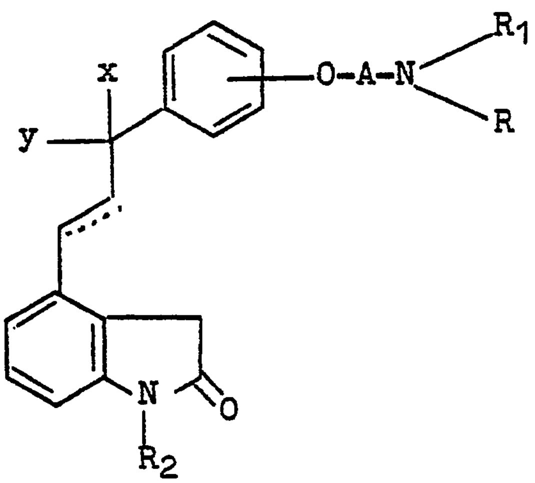

$I_F$

dans laquelle x, y, A, R, $R_1$, $R_2$ et le pointillé ont la signification déjà indiquée, que l'on isole et, si désiré, salifie.

8) Procédé pour la préparation des dérivés répondant à la formule I selon la revendication 1 et de leurs sels d'addition avec les acides minéraux ou organiques, caractérisé en ce que l'on fait réagir un produit de formule II :

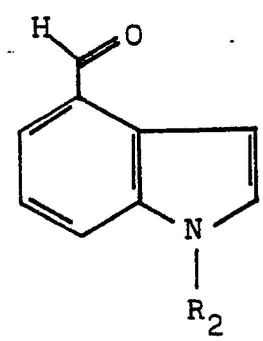

II

dans laquelle $R_2$ a la signification indiquée précédemment avec un dérivé de formule III' :

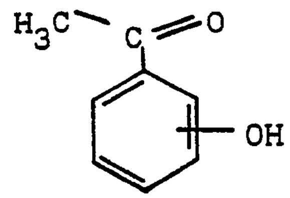

III'

pour obtenir un produit de formule IX :

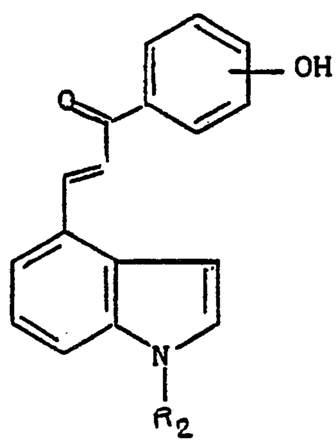

IX

dans laquelle $R_2$ a la signification déjà indiquée, que soit l'on hydrogène par action d'hydrogène gazeux en présence d'un catalyseur à base de platine ou de palladium dans un solvant tel qu'un alcanol renfermant de 1 à 5 atomes de carbone, ou par action d'hydrogène gazeux en présence de Nickel de Raney dans un solvant tel que l'acétate d'éthyle, ou par action pendant moins de 3 heures, de sodium dans l'ammoniac, dans un solvant tel que le tétrahydrofuranne, pour obtenir un produit de formule X :

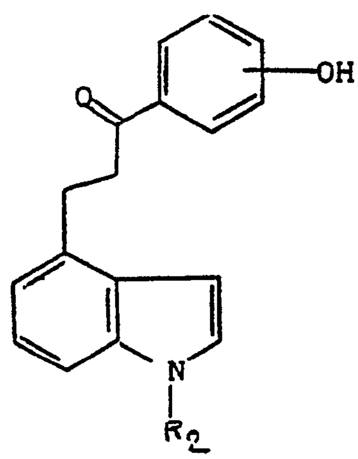

X

dans laquelle $R_2$ a la signification déjà indiquée,
que ou bien l'on réduit à l'aide d'un borohydrure ou cyanoborohydrure alcalin pour obtenir un produit de formule XI :

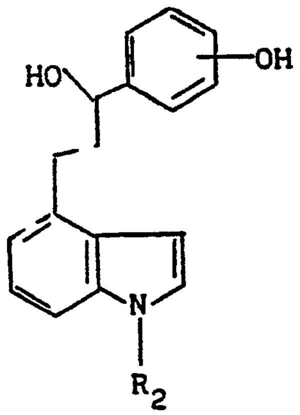

XI

dans laquelle $R_2$ a la signification déjà indiquée, que l'on réduit à l'aide de sodium dans l'ammoniac pour obtenir un produit de formule XII :

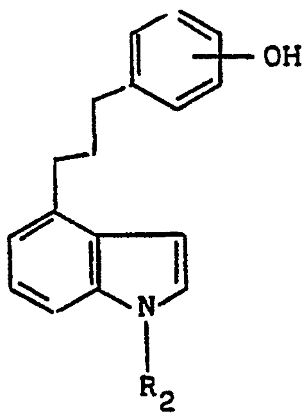

XII

dans laquelle $R_2$ a la signification déjà indiquée,
soit l'on fait réagir ledit produit de formule IX avec le complexe formé entre un borohydrure alcalin et la pyridine, pour obtenir un produit de formule XIII :

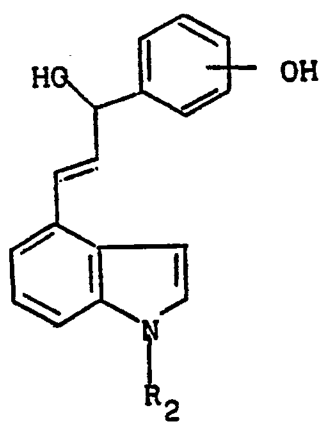

XIII

dans laquelle $R_2$ a la signification déjà indiquée, que l'on réduit à l'aide de sodium dans l'ammoniac pour obtenir un produit de formule XII, soit l'on réduit ledit produit de formule IX à l'aide de sodium dans l'ammoniac pour obtenir un produit de formule XII défini ci-dessus ou bien l'on réduit ledit produit de formule X à l'aide d'hydrazine et de lessive de potasse dans l'éthyléne glycol pour obtenir un produit de formule XII défini ci-dessus, puis soumet, si désiré, lesdits produits formules IX, X, XI, XII et XIII à l'action d'un agent d'halogénation pour obtenir un produit de formule XIV :

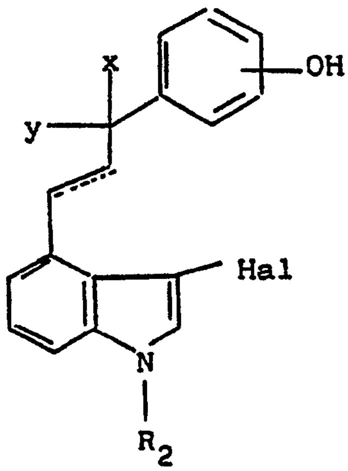

XIV

dans laquelle Hal représente un atome de brome ou de chlore, x, y, $R_2$ et le pointillé ont la signification déjà indiquée, que l'on soumet à une hydrolyse pour obtenir un produit de formule XV :

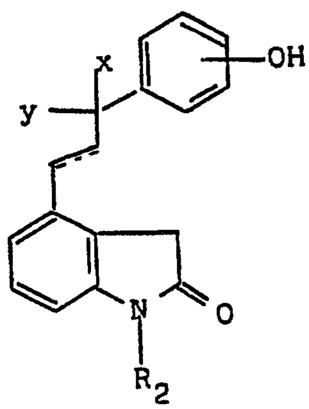

XV

dans laquelle x, y, $R_2$ et le pointillé ont la signification déjà indiquée puis fait réagir lesdits produits de formules IX, X, XI, XII, XIII et XV avec un halogénure de formule XVI :

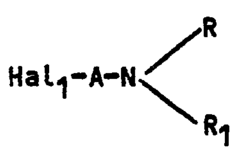

XVI

dans laquelle Hal représente un atome de chlore, de brome ou d'iode, A, R et $R_1$ ont la signification indiquée précédemment, pour obtenir un produit de formule $I_F$:

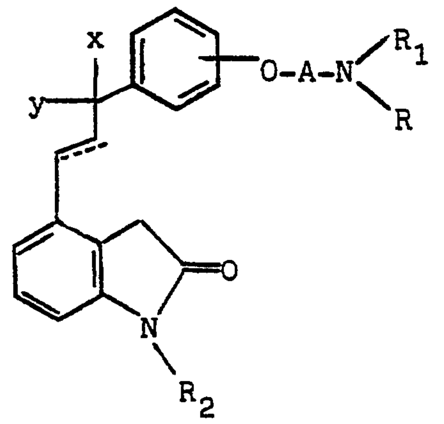

$I_F$

dans laquelle le pointillée, x, y, A, R, $R_1$ et $R_2$ ont la signification déjà indiquée, que l'on isole et, si désiré, salifie.

9) Procédé selon la revendication 7 ou 8, caractérisé en ce que l'agent d'halogénation est un N-halo succinimide.

10) Médicaments caractérisés en ce qu'ils sont constitués par les nouveaux dérivés du 4-phényl propyl indole tels que défniis par la formule I, revendication 1 ainsi que par leurs sels d'addition avec les acides pharmaceutiquement acceptables.

11) Médicaments caractérisés en ce qu'ils sont constitués par les nouveaux dérivés du 4-phényl propyl indole tels que définis à la revendication 2 ou 3, ainsi que leurs sels d'addition avec les acides pharmaceutiquement acceptables.

12) Médicaments caractérisés en ce qu'ils sont constitués par un dérivé du 4-phénylpropyl-indole, tel que défini à l'une quelconque des revendications 4, 5 ou 6 ainsi que par leurs sels d'addition avec les acides pharmaceutiquement acceptables.

13) Compositions pharmaceutiques, caractérisées en ce qu'elles renferment, à titre de principe actif, l'un au moins des médicaments tels que définis à l'une des revendications 10, 11 ou 12.

**Patentansprüche**

1. 4-Phenylpropylindolderivate sowie deren Additionssalze mit Mineral- oder organischen Säuren, dadurch gekennzeichnet, daß sie der allgemeinen Formel I

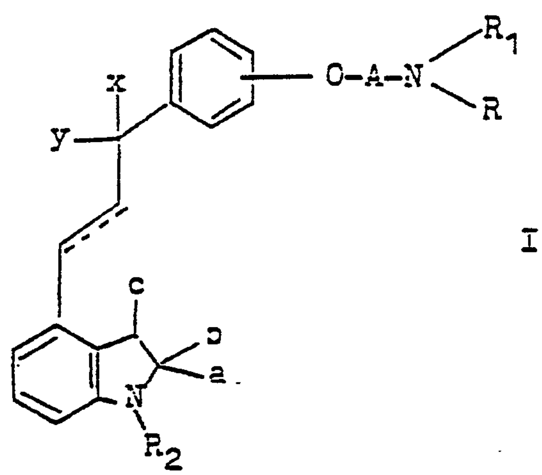

I

entsprechen, worin R und $R_1$ voneinander unabhängig für ein Wasserstoffatom, einen linearen Alkylrest mit 1 bis 5 Kohlenstoffatomen, einen verzweigten Alkylrest mit 3 bis 5 Kohlenstoffatomen, einen Cycloalkylrest mit 3 bis 7 Kohlenstoffatomen, einen Cycloalkylalkylrest mit 4 bis 7 Kohlenstoffatomen oder einen Aralkylrest mit 7 bis 12 Kohlenstoffatomen oder einen substituierten Aralkylrest mit 7 bis 12 Kohlenstoffatomen, ausgewählt unter den Benzyl- oder Phenetylresten, substituiert durch 1, 2 oder 3 Reste, ausgewählt aus der Gruppe der Halogene und der Methyl-, Ethyl-, Methoxy-, Ethoxy-, Trifluormethyl-, Methylthio-, Amino- und Nitrogruppen, stehen oder R und $R_1$ gemeinsam mit dem Stickstoffatom einen Pyrrolidino-, Piperidino-, Morpholino-, Piperazinyl-, Methylpiperazinyl-, Ethylpiperazinyl-, Propylpiperazinylring bilden; a gemeinsam mit b eine Oxofunktion bildet oder gemeinsam mit c eine Kohlenstoff-Kohlenstoff-Bindung wiedergibt; b für ein Wasserstoffatom steht oder gemeinsam mit a eine Oxofunktion bildet; c für ein Wasserstoffatom steht oder gemeinsam mit a eine Kohlenstoff-Kohlenstoff-Bindung bildet; die gestrichelte Linie die etwaige Anwesenheit einer Kohlenstoff-Kohlenstoff-Bindung anzeigt; A eine Kette $-(CH_2)_n-$ wiedergibt, in der n die Werte 2, 3, 4 oder 5 annehmen kann; $R_2$ für ein Wasserstoffatom, eine lineare Alkylgruppe mit 1 bis 5 Kohlenstoffatomen oder einen verzweigten Alkylrest mit 3 bis 5 Kohlenstoffatomen steht; x eine Hydroxylgruppe oder ein Wasserstoffatom darstellt oder gemeinsam mit y eine Oxofunktion darstellt; und y ein Wasserstoffatom wiedergibt oder gemeinsam mit x eine Oxofunktion darstellt.

2. 4-Phenylpropylindolderivate der Formel I gemäß Anspruch 1 sowie deren Additionssalze mit Mineral- oder organischen Säuren, dadurch gekennzeichnet, daß a und c gemeinsam eine Kohlenstoff-Kohlenstoff-Bindung bedeuten.

3. 4-Phenylpropylindolderivate gemäß Anspruch 2 sowie deren Additionssalze mit Mineral- oder organischen Säuren, dadurch gekennzeichnet, daß $R_1$ und $R_2$ ein Wasserstoffatom bedeuten.

4. Derivat der Formel I gemäß Anspruch 1 mit der folgenden Bezeichnung: N-[2-[2-[3-(1H-Indol-4-yl)-propyl]-phenoxy]-ethyl]-2-methyl-2-propanamin sowie dessen Additionssalze mit Mineral- oder organischen Säuren.

5. 1-[2-[3-[(1,1-Dimethylethyl)-amino]-propoxy]-phenyl]-3-(1H-indol-4-yl)-1-propanon sowie dessen Additionssalze mit Mineral- oder organischen Säuren.

6. N-[2-[2-[3-(1H-Indol-4-yl)-propyl]-phenoxy]-ethyl]-N-(1-methylethyl)-2-propanamin sowie dessen Additionssalze mit Mineral- oder organischen Säuren.

7. Verfahren zur Herstellung der 4-Phenylpropylindolderivate der Formel I gemäß Anspruch 1 sowie von deren Salzen, dadurch gekennzeichnet, daß man ein Derivat der Formel II

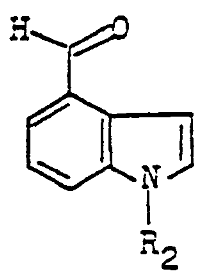

II

worin $R_2$ die angegebene Bedeutung besitzt, mit einem Derivat der Formel III

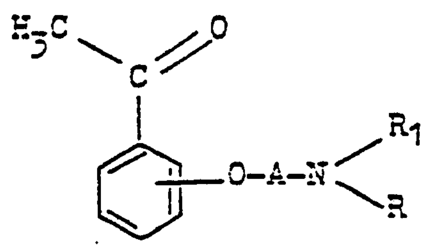

III

worin R und $R_1$ die angegebenen Bedeutungen besitzen, umsetzt, um zu einem Produkt der Formel $I_A$

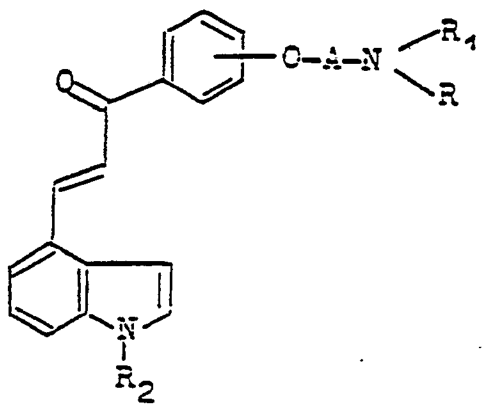

$I_A$

zu gelangen, worin R, $R_1$ und $R_2$ die angegebene Bedeutung besitzen, das man <u>entweder</u> durch Einwirken von gasförmigem Wasserstoff in Anwesenheit eines Katalysators auf Platin- oder Palladiumbasis in einem Lösungsmittel, wie einem Alkanol mit 1 bis 5 Kohlenstoffatomen, oder durch Einwirken von gasförmigem Wasserstoff in Anwesenheit von Raneynickel in einem Lösungsmittel, wie Ethylacetat, oder durch Einwirken während weniger als 3 Stunden von Natrium in Ammoniak in einem Lösungsmittel, wie Tetrahydrofuran, hydriert, um zu einem Produkt der Formel $I_B$

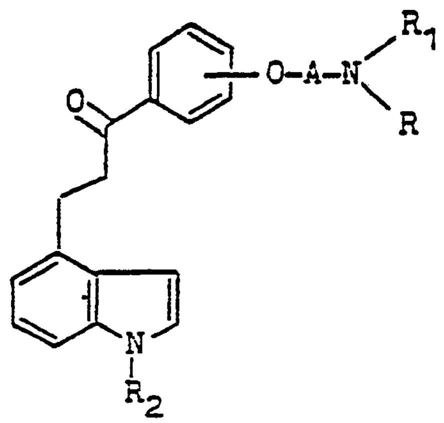

$I_B$

zu gelangen, worin A, R, $R_1$ und $R_2$ die angegebene Bedeutung besitzen, das man entweder isoliert und gewünschtenfalls in ein Salz überführt, oder mit Hilfe eines Alkaliborhydrids oder-cyanoborhydrids reduziert, um zu einem Produkt der Formel $I_C$

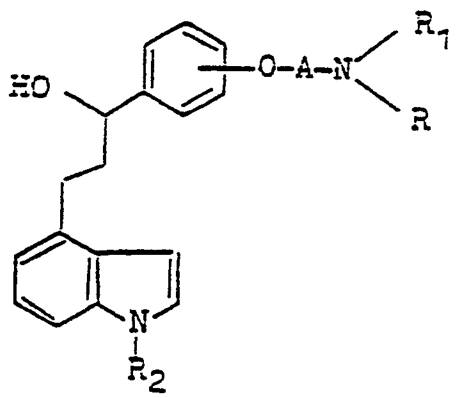

$I_C$

zu gelangen, worin A, R, $R_1$ und $R_2$ die angegebene Bedeutung besitzen, das man entweder isoliert und gewünschtenfalls in ein Salz überführt oder mit Hilfe von Natrium in Ammoniak reduziert, um zu einem Produkt der Formel $I_D$

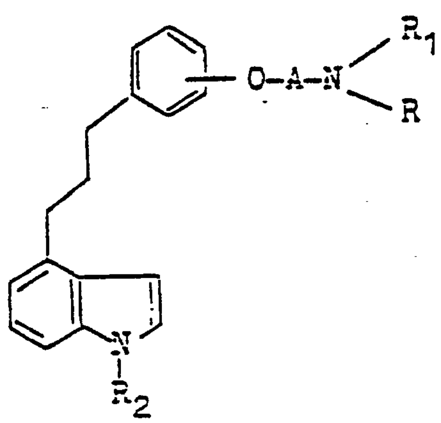

$I_D$

zu gelangen, worin A, R, $R_1$ und $R_2$ die angegebene Bedeutung besitzen, das man isoliert und gewünschtenfalls in ein Salz überführt, oder das Produkt der Formel $I_A$ mit dem Komplex, gebildet zwischen einem Alkaliborhydrid und Pyridin, umsetzt, um zu einem Produkt der Formel $I_E$

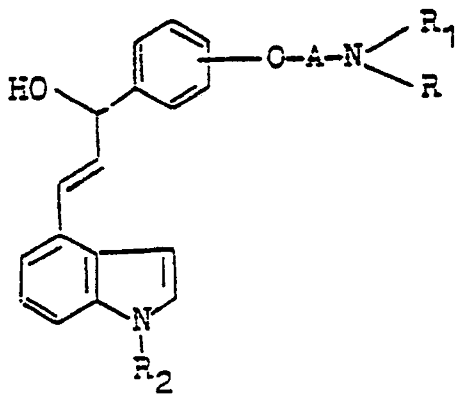

$I_E$

zu gelangen, worin A, R, $R_1$ und $R_2$ die angegebene Bedeutung besitzen, das man entweder isoliert oder mit Hilfe von Natrium in Ammoniak reduziert, um zu einem Produkt der Formel $I_D$ zu gelangen, das man isoliert und gewünschtenfalls in ein Salz überführt, oder das Produkt der Formel $I_A$ mit Hilfe von Natrium in Ammoniak reduziert, um zu einem Produkt der Formel $I_D$ zu gelangen, das man isoliert und gewünschtenfalls in ein Salz überführt, anschließend gewünschtenfalls die Produkte der Formel $I_A$, $I_B$, $I_C$, $I_D$ und $I_E$ der Einwirkung eines Halogenierungsmittels unterzieht, um zu einem Produkt der Formel IV

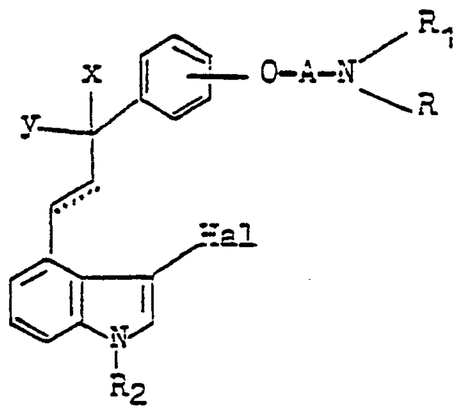

IV

zu gelangen, worin Hal für ein Brom- oder Chloratom steht und x, y, A, R, $R_1$, $R_2$ und die gestrichelte Linie die angegebene Bedeutung besitzen, das man einer Hydrolyse unterzieht, um zu einem Produkt der Formel $I_F$

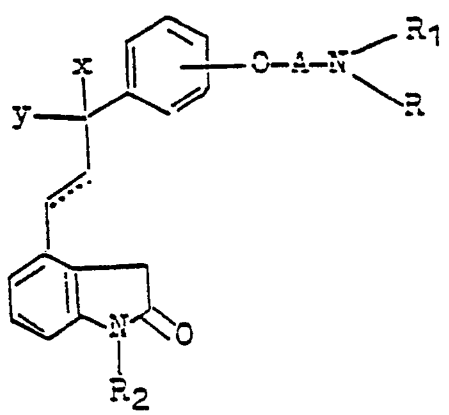

$I_F$

zu gelangen, worin x, y, A, R, $R_1$ $R_2$ und die gestrichelte Linie die angegebene Bedeutung besitzen, das man isoliert und gewünschtenfalls in ein Salz überführt.

8. Verfahren zur Herstellung der Derivate der Formel I gemäß Anspruch 1 und deren Additionssalzen mit Mineral- oder organischen Säuren, dadurch gekennzeichnet, daß man ein Produkt der Formel II

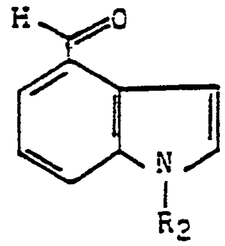

II

worin $R_2$ die angegebene Bedeutung besitzt, mit einem Derivat der Formel III'

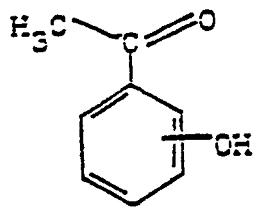

III'

umsetzt, um zu einem Produkt der Formel IX

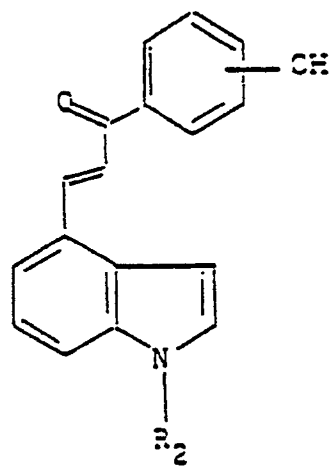

IX

zu gelangen, worin $R_2$ die angegebene Bedeutung besitzt, das man entweder durch Einwirken von gasförmigem Wasserstoff in Anwesenheit eines Katalysators auf Platin- oder Palladiumbasis in einem Lösungsmittel, wie einem Alkanol mit 1 bis 5 Kohlenstoffatomen, oder durch Einwirken von gasförmigem Wasserstoff in Anwesenheit von Raneynickel in einem Lösungsmittel, wie Ethylacetat, oder durch Einwirken während weniger als 3 Stunden von Natrium in Ammoniak in einem Lösungsmittel, wie Tetrahydrofuran, hydriert, um zu einem Produkt der Formel X

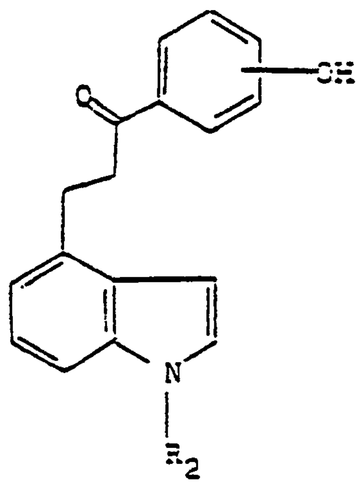

X

zu gelangen, worin $R_2$ die angegebene Bedeutung besitzt, das man entweder mit einem Alkaliborhydrid oder -cyanoborhydrid reduziert, um zu einem Produkt der Formel XI

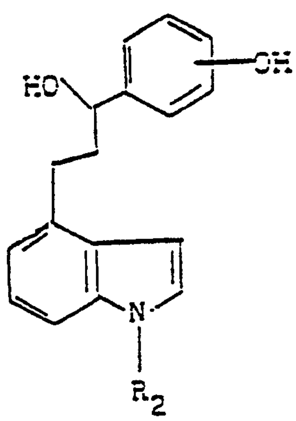

XI

zu gelangen, worin $R_2$ die angegebene Bedeutung besitzt, das man mit Natrium in Ammoniak reduziert, um zu einem Produkt der Formel XII

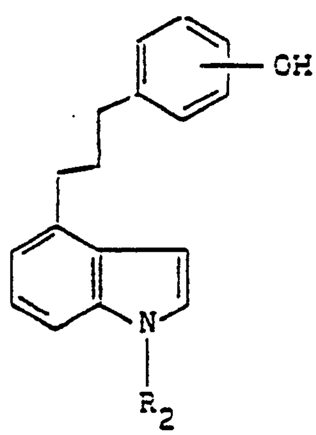

XII

zu gelangen, worin $R_2$ die angegebene Bedeutung besitzt, <u>oder</u> das Produkt der Formel IX mit dem Komplex zwischen einem Alkaliborhydrid und Pyridin umsetzt, um zu einem Produkt der Formel XIII

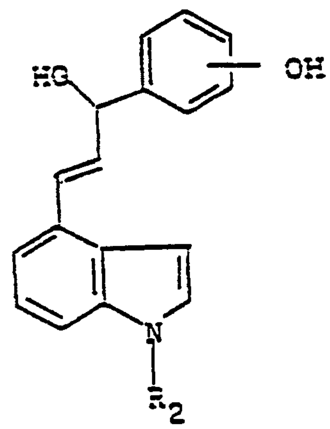

XIII

zu gelangen, worin $R_2$ die angegebene Bedeutung besitzt, das man mit Natrium in Ammoniak reduziert, um zu einem Produkt der Formel XII zu gelangen, <u>oder</u> das Produkt der Formel IX mit Natrium in Ammoniak reduziert, um zu einem Produkt der vorstehenden Formel XII zu gelangen, oder das Produkt der Formel X mit Hydrazin und Kalilauge in Ethylenglykol reduziert, um zu dem vorstehend definierten Produkt der Formel XII zu gelangen, hiernach gewünschtenfalls die Produkte der Formeln IX, X, XI, XII und XIII der Einwirkung eines Halogenierungsmittels unterzieht, um zu einem Produkt der Formel XIV

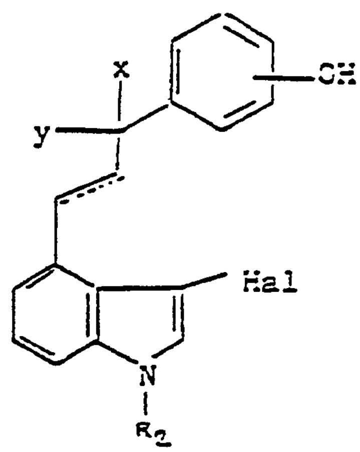

XIV

zu gelangen, worin Hal für ein Brom- oder Chloratom steht und x, y, $R_2$ und die gestrichelte Linie die angegebene Bedeutung besitzen, das man einer Hydrolyse unterzieht, um zu einem Produkt der Formel XV

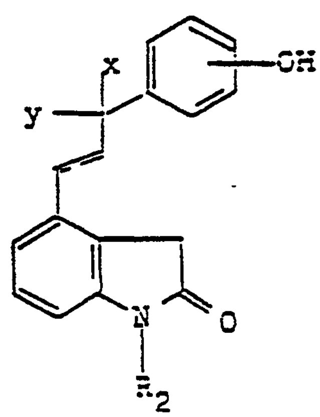

XV

zu gelangen, worin x, y, $R_2$ und die gestrichelte Linie die angegebene Bedeutung besitzen, hiernach die Produkte der Formeln IX, X, XI, XII, XIII und XV mit einem Halogenid der Formel XVI

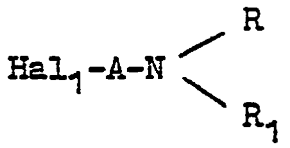

XVI

worin $Hal_1$ für ein Chlor-, Brom- oder Jodatom steht und A, R und $R_1$ die angegebene Bedeutung besitzen, umsetzt, um zu einem Produkt der Formel $I_F$

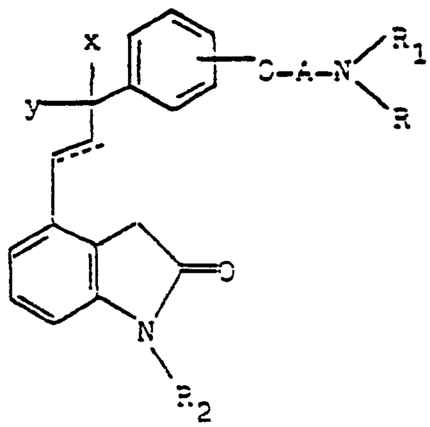

$I_F$

zu gelangen, worin die gestrichelte Linie, x, y, A, R, $R_1$ und $R_2$ die angegebene Bedeutung besitzen, das man isoliert und gewünschtenfalls in ein Salz überführt.

9. Verfahren gemäß Anspruch 7 oder 8, dadurch gekennzeichnet, daß das Halogenierungsmittel N-Halosuccinimid ist.

10. Arzneimittel, dadurch gekennzeichnet, daß sie aus den neuen 4-Phenylpropylindolderivaten der Formel I gemäß Anspruch 1 sowie aus deren Additionssalzen mit pharmazeutisch verträglichen Säuren bestehen.

11. Arzneimittel, dadurch gekennzeichnet, daß sie aus den neuen 4-Phenylpropylindolderivaten gemäß Anspruch 2 oder 3 sowie aus deren Additionssalzen mit pharmazeutisch verträglichen Säuren bestehen.

12. Arzneimittel, dadurch gekennzeichnet, daß sie aus einem 4-Phenylpropylindolderivat gemäß einem der Ansprüche 4, 5 oder 6 sowie aus deren Additionssalzen mit pharmazeutisch verträglichen Säuren bestehen.

13. Pharmazeutische Zusammensetzungen, dadurch gekennzeichnet, daß sie als Wirkstoff zumindest eines der Arzneimittel gemäß einem der Ansprüche 10, 11 oder 12 enthalten.

**Claims**

1. The derivatives of 4-phenylpropyl-indole, as well as their addition salts with mineral or organic acids, characterized in that they correspond to the general formula (I):

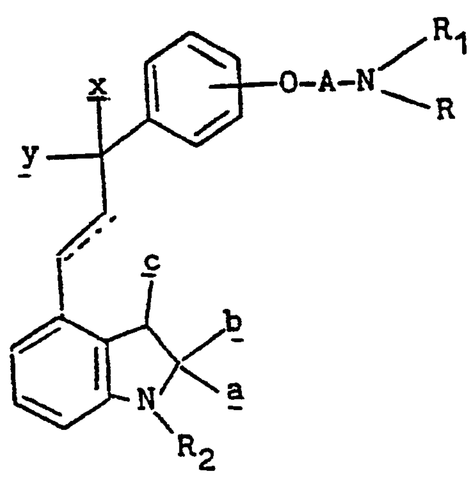

(I)

in which R and $R_1$ represent, independently from one another, a hydrogen atom, a linear alkyl radical containing from 1 to 5 carbon atoms, a branched alkyl radical containing from 3 to 5 carbon atoms, a cycloalkyl radical containing from 3 to 7 carbon atoms, a cycloalkylalkyl radical containing from 4 to 7 carbon atoms, or a substituted aralkyl radical containing from 7 to 12 carbon atoms, chosen from benzyl or phenethyl radicals substituted by 1, 2 or 3 radicals chosen from the group constituted by halogens and the following radicals: methyl, ethyl, methoxy, ethoxy, trifluoromethyl, methylthio, amino and nitro; or R and $R_1$ form, together with the nitrogen atom, one of the following rings: pyrrolidino, morpholino, piperazinyl, methyl piperazinyl, ethyl piperazinyl, propyl piperazinyl, a represents, together with b, an oxo function, or represents, together with c, a carbon-carbon bond, b represents a hydrogen atom, or together with a on oxo function, c represents a hydrogen atom, or together with a represents a carbon-carbon bond, the dotted line represents the possible presence of a carbon-carbon bond, A represents a $-(CH_2)_n-$ chain in which n can have the values 2, 3, 4 or 5, $R_2$ represents a hydrogen atom, a linear alkyl radical containing from 1 to 5 carbon a toms or a branched alkyl radical containing from 3 to 5 carbon atoms, x represents a hydroxyl radical or a hydrogen atom, or together with y an oxo function, and y represents a hydrogen atom or, together with x an oxo function.

2. The derivatives of 4-phenylpropyl-indole as defined by the formula (I) of claim 1, as well as their addition salts with mineral or organic acids, characterized in that a and c together represent a carbon-carbon bond.

3. The derivatives of 4-phenylpropyl-indole according to claim 2, as well as their addition salts with mineral or organic acids, characterized in that $R_1$ and $R_2$ each represents a hydrogen atom.

4. The derivative of formula (I), according to claim 1, of which the name follows:
N-[2-[2-[3-(1H-indol-4-yl)propyl]phenoxy]ethyl]-2-methyl-2-propanamine, as well as its addition salts with mineral or organic acids.

5. 1-[2-[3-[(1,1-dimethylethyl)amino]propoxy]phenyl]-3-(1H-indol-4-yl)-1-propanone, as well as its addition salts with mineral or organic acids.

6. N-[2-[2-[3-(1H-indol-4-yl)propyl]phenoxy]ethyl]-N-(1-methylethyl)-2-propanamine, as well as its addition salts with mineral or organic acids.

7. Process for the preparation of derivatives of 4-phenylpropyl-indole as defined by the formula (I) of claim 1, as well as their salts, characterized in that a derivative of formula (II):

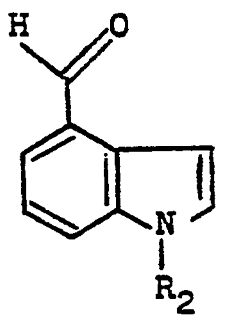

(II)

in which $R_2$ has the significance already indicated, is reacted with a derivative of formula (III):

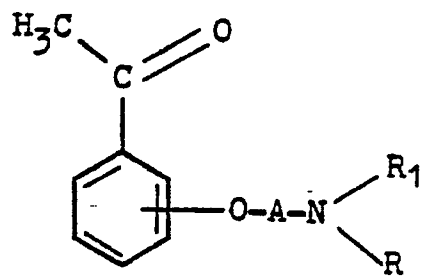

(III)

in which R and $R_1$ have the significance already indicated, so as to obtain a product of formula ($I_A$):

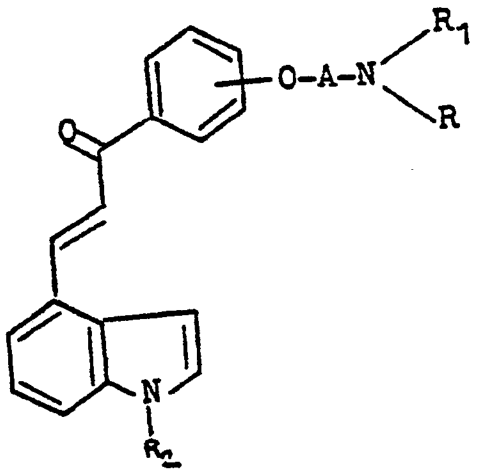

$(I_A)$

in which R, $R_1$ and $R_2$ have the significance already indicated, which is either hydrogenated by the action of gaseous hydrogen in the presence of a catalyst based on platinum or palladium in a solvent such as an alkanol containing from 1 to 5 carbon atoms, or by the action of gaseous hydrogen in the presence of Raney's Nickel in a solvent such as ethyl acetate, or by the action, for less than 3 hours, of sodium in ammonia, in a solvent such as tetrahydrofuran, so as to obtain a product of formula $(I_B)$:

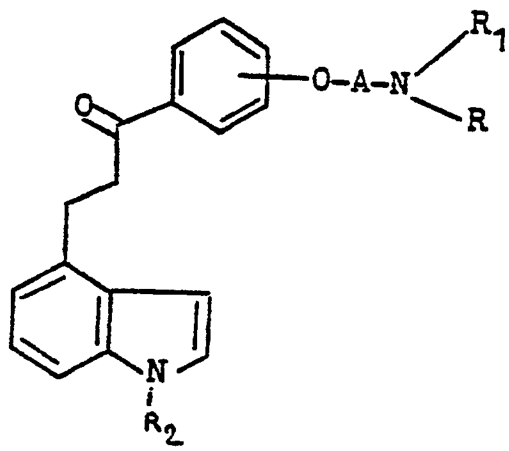

$(I_B)$

in which A, R, $R_1$ and $R_2$ have the significance already indicated, which is either isolated and, if desired, salified, or reduced with an alkaline borohydride or a cyanoborohydride so as to obtain a product of formula $I_C$

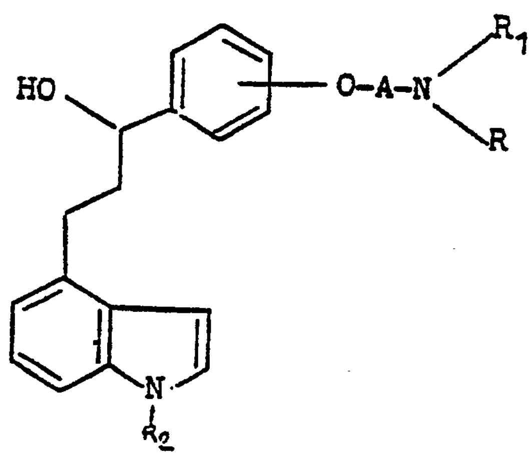

$(I_C)$

in which A, R, $R_1$ and $R_2$ have the significance already indicated, which is either isolated and, if desired, salified, or reduced with sodium in ammonia so as to obtain a product of formula ($I_D$):

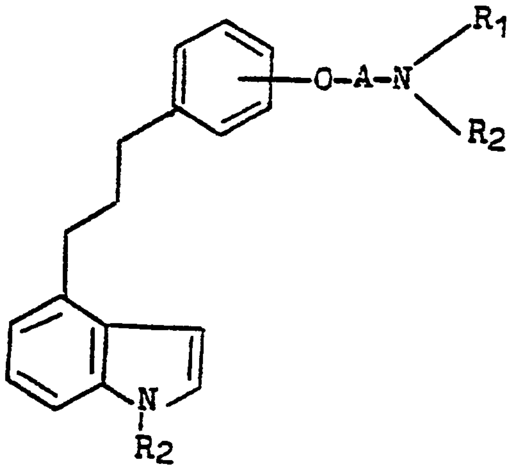

($I_D$)

in which A, R, $R_1$ and $R_2$ have the significance already indicated, which is isolated and, if desired, salified, or the said product of formula ($I_A$) is reacted with the complex formed between an alkaline borohydride and pyridine, so as to obtain a product of formula ($I_E$):

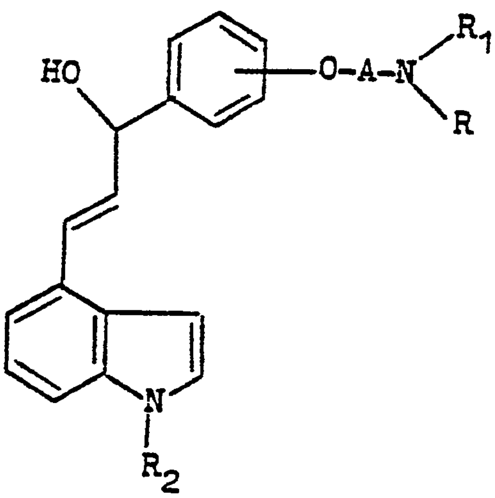

($I_E$)

in which A, R, $R_1$ and $R_2$ have the significance already indicated, which is either isolated, or reduced using sodium in ammonia so as to obtain a product of formula ($I_D$) which is isolated and, if desired, salified, or the said product of formula ($I_A$) is reduced with sodium in ammonia so as to obtain a product of formula ($I_D$) which is isolated and, if desired, salified, then, if desired, the said products of formulae ($I_A$), ($I_B$), ($I_C$), ($I_D$) and ($I_E$) are submitted to the action of a halogenation agent so as to obtain a product of formula (IV):

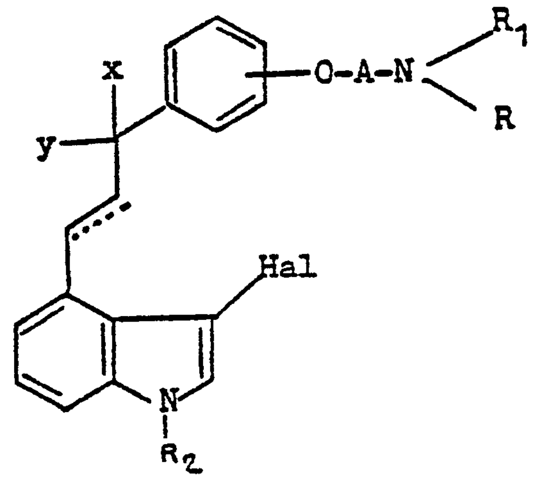

(IV)

in which Hal represents a bromine or chlorine atom, and x, y, A, R, $R_1$, $R_2$ and the dotted line have the significance already indicated, which is submitted to an hydrolysis so as to obtain a product of formula ($I_F$):

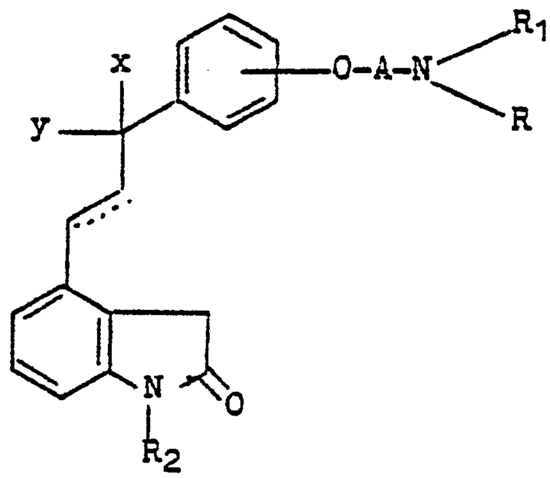

($I_F$)

in which x, y, A, R, $R_1$, $R_2$ and the dotted line have the significance already indicated, which is isolated and, if desired, salified.

8. Process for the preparation of derivatives corresponding to formula (I) according to claim 1 and of their addition salts with mineral or organic acids, characterized in that a product of formula (II):

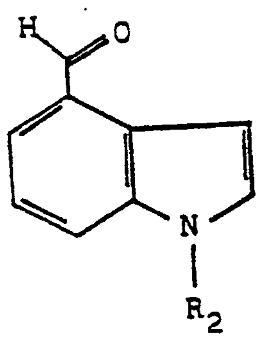

(II)

in which $R_2$ has the previously indicated significance, is reacted with a derivative of formula (III'):

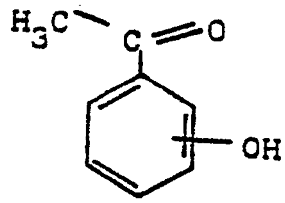

(III')

so as to obtain a product of formula (IX):

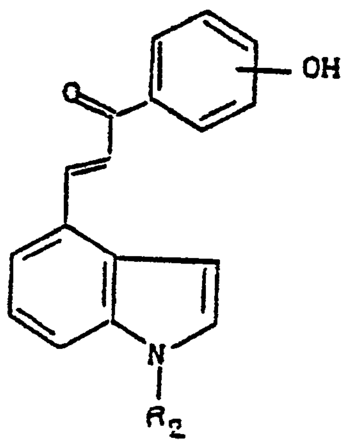

(IX)

in which $R_2$ has the significance already indicated, which is either hydrogenated by the action of gaseous hydrogen in the presence of a catalyst based on platinum or palladium in a solvent such as an alkanol containing from 1 to 5 carbon atoms, or by the action of gaseous hydrogen in the presence of Raney's Nickel in a solvent such as ethyl acetate or by the action, for less than 3 hours, of sodium in ammonia in a solvent such as tetrahydrofuran, so as to obtain a product of formula (X):

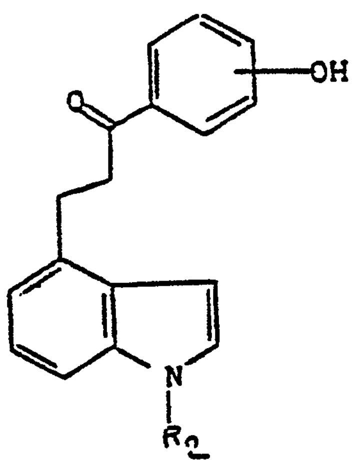

(X)

in which $R_2$ has the significance already indicated, or which is reduced using an alkaline borohydride or cyanoborohydride so as to obtain a product of formula (XI):

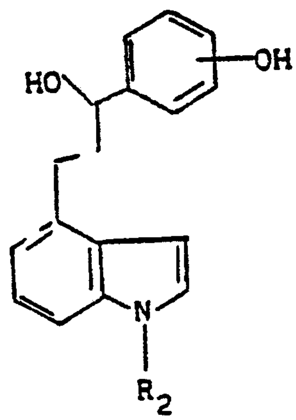

(XI)

in which $R_2$ has the significance already indicated, which is reduced using sodium in ammonia so as to obtain a product of formula (XII):

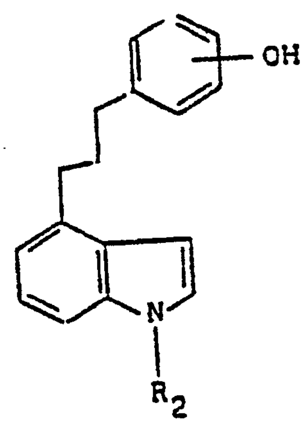

(XII)

in which $R_2$ has the significance already indicated, or the said product of formula (IX) is reacted with the complex formed between an alkaline borohydride and pyridine, so as to obtain a product of formula (XIII):

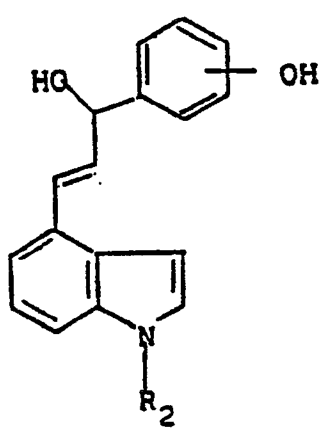

(XIII)

in which $R_2$ has the significance already indicated, which is reduced using sodium in ammonia so as to obtain a product of formula (XII), or the said product of formula (IX) is reduced using sodium in ammonia so as to obtain a product of formula (XII) defined above, or the said product of formula (X) is reduced using hydrazine and potassium hydroxide solution in ethylene glycol, so as to obtain a product of formula (XII) defined above, then the said products of formulae (IX), (X), (XI), (XII) and (XIII) are submitted, if desired, to the action of a halogenation agent so as to obtain a product of formula (XIV):

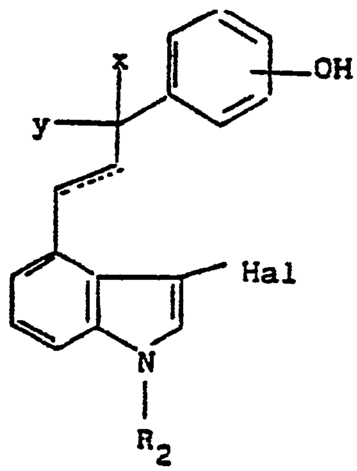

(XIV)

in which Hal represents a bromine or chlorine atom and x, y, $R_2$ and the dotted line have the significance already indicated, which is submitted to a hydrolysis so as to obtain a product of formula (XV):

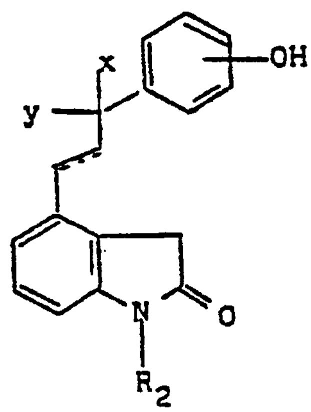

(XV)

in which x, y, $R_2$ and the dotted line have the significance already indicated, then the said products of formulae (IX), (X), (XI), (XII), (XIII) and (XV) are reacted with a halide of formula (XVI):

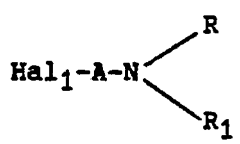

(XVI)

in which $Hal_1$ represents a chlorine, bromine or iodine atom and A, R and $R_1$ have the previously indicated significance, so as to obtein a product of formula ($I_F$):

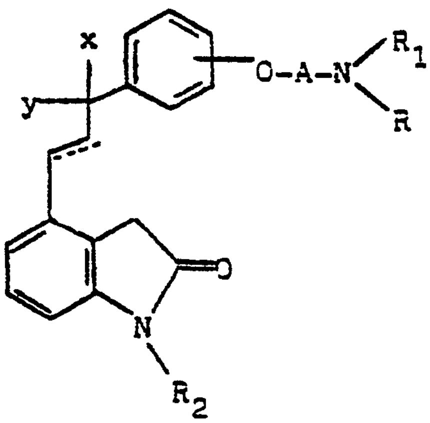

($I_F$)

in which the dotted line, x, y, A, R, $R_1$ and $R_2$ have the significance already indicated, which is isolated and, if desired, salified.

9. Process according to claim 7 or 8, characterized in that the halogenation agent is an N-halo succinimide.

10.Medicaments characterized in that they are constituted by the new derivatives of 4-phenylpropyl-indole as defined by formula (I), claim 1, as well as by their addition salts with pharmaceutically acceptable acids.

11. Medicaments characterized in that they are constituted by the new derivatives of 4-phenylpropyl-indole as defined in claim 2 or 3, as well as by their addition salts with pharmaceutically acceptable acids.

12. Medicaments characterized in that they are constituted by a derivative of 4-phenylpropyl-indole, as defined by any one of claims 4, 5 or 6, as well as by their addition salts with pharmaceutically acceptable acids.

13. Pharmaceutical compositions, characterized in that they contain, as active principle, at least one of the medicaments as defined in one of claims 10, 11 or 12.